(19) Europäisches Patentamt
European Patent Office
Office européen des brevets

(11) EP 0 246 534 B1

(12) EUROPÄISCHE PATENTSCHRIFT

(45) Veröffentlichungstag und Bekanntmachung des Hinweises auf die Patenterteilung:
**15.01.1997 Patentblatt 1997/03**

(51) Int. Cl.$^6$: **C07D 207/28**, C07D 241/08, A61K 31/40

(21) Anmeldenummer: **87106840.9**

(22) Anmeldetag: **12.05.1987**

# (54) Enantiomerenreine 4,5-disubstituierte gamma-Butyrolactame, Verfahren zu ihrer Herstellung und ihre Verwendung

Optically pure 4,5-disubstituted gamma-butyrolactame enantiomers, process for their preparation and their use

Enantiomères optiquement purs de 4,5-disubstitué-gamma-butyrolactame, leur procédé de préparation et utilisation

(84) Benannte Vertragsstaaten:
**AT BE CH DE ES FR GB IT LI NL SE**

(30) Priorität: **21.05.1986 DE 3616989**

(43) Veröffentlichungstag der Anmeldung:
**25.11.1987 Patentblatt 1987/48**

(73) Patentinhaber:
- **BAYER AG**
  **51368 Leverkusen (DE)**
- **CHINESE ACADEMY OF MEDICAL SCIENCES**
  **Beijing (CN)**

(72) Erfinder: **Hartwig, Wolfgang, Dr.**
**Appartment 1604 Chicago Illinois 60615 (US)**

(74) Vertreter: **Dänner, Klaus, Dr. et al**
**Bayer AG**
**Konzernverwaltung RP**
**Patente Konzern**
**51368 Leverkusen (DE)**

(56) Entgegenhaltungen:
**EP-A- 0 172 514** **DE-A- 3 632 589**

- **CHEMISCHE BERICHTE, Jahrgang 100, Heft 4, 1967, Seiten 1137-1143; F. ZYMALKOWSKI et al.: "Eine stereoselektive Synthese von cis- und trans-3-phenyl-2-carboxy-pyrrolidon-(5)"**
- **SYNTHESIS, Nr. 9, September 1986, Seiten 737-740; U. SCHÖLLKOPF et al.: "Assymmetric synthesis via heterocyclic intermediates"**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 246 534 B1

Printed by Rank Xerox (UK) Business Services
2.13.13/3.4

## Beschreibung

Die Erfindung betrifft enantiomerenreine 4,5-disubstituierte γ-Butyrolactame, Verfahren zu ihrer Herstellung und ihre Verwendung als Zwischenprodukte für pharmazeutische Wirkstoffe.

Die Erfindung stellt enantiomerenreine 4,5-disubstituierte γ-Butyrolactame der allgemeinen Formel (I)

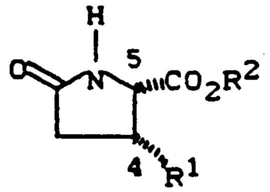

(I),

in welcher

$R^1$ - für mit 6 bis 14 Kohlenstoffatomen steht, das bis zu 5-fach gleich oder verschieden substituiert sein kann durch Alkyl, Alkoxy, Alkylthio mit jeweils bis zu 8 Kohlenstoffatomen, durch Aryl, Aryloxy, Arylthio mit jeweils 6 bis 12 Kohlenstoffatomen, durch Aralkyl mit 7 bis 14 Kohlenstoffatomen durch Halogen, Trifluormethyl, Trifluormethoxy, Difluormethoxy, Trifluormethylthio, Nitro, Cyano, Carboxy, Alkoxycarbonyl mit bis zu 8 Kohlenstoffatomen, Sulfo, Phenylsulfonyl, Tolylsulfonyl, Alkylsulfonyl mit bis zu 8 Kohlenstoffatomen, Hydroxy oder durch eine Gruppe der Formel

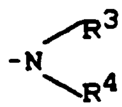

,

worin

$R^3$ und $R^4$ gleich oder verschieden sind und Wasserstoff, Alkyl mit bis zu 8 Kohlenstoffatomen, Aryl mit 6 bis 12 Kohlenstoffatomen, Aralkyl mit 7 bis 14 Kohlenstoffatomen, Acyl mit bis zu 7 Kohlenstoffatomen, Alkylsulfonyl mit bis zu 6 Kohlenstoffatomen, Phenylsulfonyl oder Tolylsulfonyl bedeuten,

oder

- für einen heterocyclischen Ring der Reihe Furyl, Thienyl, Pyridyl, Pyrimidyl, Pyrazinyl, Pyridazinyl, Chinolyl oder Isochinolyl steht, wobei diese Ringe bis zu 3-fach gleich oder verschieden substituiert sein können durch Alkyl, Alkoxy, Alkylthio mit jeweils bis zu 6 Kohlenstoffatomen, Halogen, Phenyl, Nitro, Cyano oder durch eine Gruppe der Formel

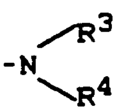

,

worin
$R^3$ und $R^4$ die oben angegebene Bedeutung haben,

oder

- für geradkettiges, verzweigtes oder cyclisches Alkyl oder Alkenyl mit bis zu 10 Kohlenstoffatomen steht, das substituiert sein kann durch Halogen, Aryl mit 6 bis 14 Kohlenstoffatomen, Furyl, Thienyl, Pyridyl, Pyrimidyl, Pyrazinyl, Pyridazinyl, Chinolyl, Isochinolyl, Hydroxy, Alkoxy, Alkylthio mit jeweils bis zu 6 Kohlenstoffatomen, Carboxy, Alkoxycarbonyl mit bis zu 6 Kohlenstoffatomen, Sulfo, Alkylsulfonyl mit bis zu 6 Kohlenstoffatomen, Phenylsulfonyl, Tolysulfonyl oder durch eine Gruppe der Formel

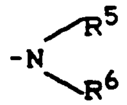 ,

worin

$R^5$ und $R^6$ gleich oder verschieden sind und Wasserstoff, Alkyl mit bis zu 8 Kohlenstoffatomen, Aryl mit 6 bis 12 Kohlenstoffatomen, Aralkyl mit 7 bis 14 Kohlenstoffatomen, Acyl mit bis zu 7 Kohlenstoffatomen, Alkylsulfonyl mit bis zu 6 Kohlenstoffatomen, Phenylsulfonyl oder Tolylsulfonyl bedeuten,
oder worin

$R^5$ und $R^6$ gemeinsam mit dem Stickstoffatom einen Ring aus der Reihe Pyrrolidino, Piperidino, Piperazino, Morpholino oder Thiomorpholino bilden und wobei dieser Ring substituiert sein kann durch Alkyl mit bis zu 4 Kohlenstoff-Atomen oder durch Phenyl

und

$R^2$ - für geradkettiges, verzweigtes oder cyclisches Alkyl mit bis zu 8 Kohlenstoffatomen steht,

in welchen das Ringkohlenstoffatom in 5-Stellung S-Konfiguration hat und in welchen der Substituent $R^1$ in 4-Stellung zum Substituenten $COOR^2$ in 5-Stellung cis konfiguriert ist, zur Verfügung.

Bevorzugte Verbindungen der allgemeinen Formel (I) sind solche,
in welchen

$R^1$ - für Phenyl oder Naphthyl steht, das bis zu 3-fach gleich oder verschieden substituiert sein kann durch Alkyl, Alkoxy mit jeweils bis zu 6 Kohlenstoffatomen, Methylthio, Phenyl, Phenoxy, Benzyl, Fluor, Chlor, Brom, Iod, Trifluormethyl, Trifluormethoxy, Difluormethoxy, Trifluormethylthio, Nitro, Cyano, Alkoxycarbonyl mit bis zu 6 Kohlenstoffatomen, Phenylsulfonyl, Tolylsulfonyl, Alkylsulfonyl mit bis zu 6 Kohlenstoffatomen, Hydroxy oder durch eine Gruppe der Formel

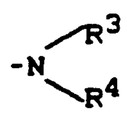 ,

in welcher

$R^3$ und $R^4$ gleich oder verschieden sind und Wasserstoff, Alkyl mit bis zu 6 Kohlenstoffatomen, Phenyl, Benzyl, Acetyl, Ethylcarbonyl, Benzoyl, Alkylsulfonyl mit bis zu 4 Kohlenstoffatomen, Tolylsulfonyl oder Phenylsulfonyl bedeuten,

oder

- für einen heterocyclischen Ring der Reihe Furyl, Thienyl, Pyridyl, Pyrimidyl, Chinolyl oder Isochinolyl steht, wobei diese Ringe substituiert sein können durch Alkyl oder Alkoxy mit jeweils bis zu 4 Kohlenstoffatomen, durch Fluor, Chlor, Brom, Nitro, Cyano oder
durch eine Gruppe der Formel

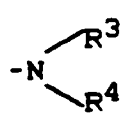 ,

worin
$R^3$ und $R^4$ die oben angegebene Bedeutung haben,

oder

- für geradkettiges, verzweigtes oder cyclisches Alkyl mit bis zu 8 Kohlenstoffatomen steht, das substituiert sein kann durch Fluor, Chlor, Brom, Phenyl, Furyl, Thienyl, Pyridyl, Pyrimidyl, Chinolyl, Isochinolyl, Hydroxy, Alkoxy oder Alkylthio mit bis zu 4 Kohlenstoffatomen, Alkoxycarbonyl mit bis zu 4 Kohlenstoffatomen, Alkylsulfonyl mit bis zu 4 Kohlenstoffatomen, Phenylsulfonyl, Tolysulfonyl oder durch eine Gruppe der Formel

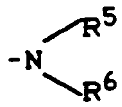 ,

worin

$R^5$ und $R^6$ gleich oder verschieden sind und Wasserstoff, Alkyl mit bis zu 6 Kohlenstoffatomen, Phenyl, Benzyl, Acetyl, Ethylcarbonyl, Benzoyl Alkylsulfonyl mit bis zu 4 Kohlenstoffatomen, Tolylsulfonyl oder Phenylsulfonyl bedeuten,

oder in welcher

$R^5$ und $R^6$ gemeinsam mit dem Stickstoffatom einen Ring aus der Reihe Pyrrolidino, Piperidino, N-Methyl- oder N-Phenylpiperazino oder Morpholino bilden

und

$R^2$ - für geradkettiges oder verzweigtes Alkyl mit bis zu 6 Kohlenstoffatomen steht,

und in welchen das Ringkohlenstoffatom in 5-Stellung S-Konfiguration hat und in welchen der Substituent $R^1$ in 4-Stellung zum Substituenten $COOR^2$ in 5-Stellung cis konfiguriert ist.

Besonders bevorzugt sind solche Verbindungen der allgemeinen Formel (I) in welchen

$R^1$ - für Phenyl steht, das substituiert sein kann durch Alkyl oder Alkoxy mit bis zu 4 Kohlenstoffatomen, durch Fluor, Chlor, Brom, Trifluormethyl, Trifluormethoxy, Trifluormethylthio, Nitro, Cyano, Alkoxycarbonyl mit bis zu 4 Kohlenstoffatomen, Phenylsulfonyl, Tolylsulfonyl, Alkylsulfonyl mit bis zu 4 Kohlenstoffatomen, Hydroxy oder durch eine Gruppe der Formel

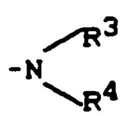 ,

in welcher
$R^3$ und $R^4$ gleich oder verschieden sind und Wasserstoff mit bis zu 4 Kohlenstoffatomen, Phenyl, Benzyl oder Acetyl bedeuten,

oder

- für Furyl, Thienyl oder Pyridyl steht, oder
- für geradkettiges, verzweigtes oder cyclisches Alkyl mit bis zu 6 Kohlenstoffatomen steht, das substituiert sein kann durch Fluor, Chlor, Brom, Phenyl, Thienyl, Pyridyl, Furyl oder Alkoxy mit bis zu 4 Kohlenstoffatomen

und

$R^2$ - für geradkettiges oder verzweigtes Alkyl mit bis zu 4 Kohlenstoffatomen steht,

in welchen das Ringkohlenstoffatom in 5-Stellung S-Konfiguration hat und in welchen der Substituent $R^1$ in 4-Stellung zum Substituenten $COOR^2$ in 5-Stellung cis konfiguriert ist.

Ebenso wurde ein Verfahren zur Herstellung enantiomerenreiner 4,5-disubstituierter γ-Butyrolactame der allgemeinen Formel (I) in welcher

$R^1$ - für Aryl mit 6 bis 12 Kohlenstoffatomen steht, das bis zu 5-fach gleich oder verschieden substituiert sein kann durch Alkyl, Alkoxy, Alkylthio mit jeweils bis zu 8 Kohlenstoffatomen, durch Aryl, Aryloxy, Arylthio mit jeweils 6 bis 12 Kohlenstoffatomen, durch Aralkyl mit 7 bis 14 Kohlenstoffatomen durch Halogen, Trifluormethyl, Trifluormethoxy, Difluormethoxy, Trifluormethylthio, Nitro, Cyano, Carboxy, Alkoxycarbonyl mit bis zu 8 Kohlenstoffatomen, Sulfo, Phenylsulfonyl, Tolysulfonyl, Alkylsulfonyl mit bis zu 8 Kohlenstoffatomen, Hydroxy oder durch eine Gruppe der Formel

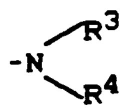 ,

worin

$R^3$ und $R^4$ gleich oder verschieden sind und Wasserstoff, Alkyl mit bis zu 8 Kohlenstoffatomen, Aryl mit 6 bis 12 Kohlenstoffatomen, Aralkyl mit 7 bis 14 Kohlenstoffatomen, Acyl mit 2 bis 7 Kohlenstoffatomen, Alkylsulfonyl mit bis zu 6 Kohlenstoffatomen, Phenylsulfonyl oder Tolylsulfonyl bedeuten,

oder

- für einen heterocyclischen Ring der Reihe Furyl, Thienyl, Pyridyl, Pyrimidyl, Pyrazinyl, Pyridazinyl, Chinolyl oder Isochinolyl steht, wobei diese Ringe bis zu 3-fach gleich oder verschieden substituiert sein können durch Alkyl, Alkoxy, Alkylthio mit jeweils bis zu 6 Kohlenstoffatomen, Halogen, Phenyl, Nitro, Cyano oder
  durch eine Gruppe der Formel

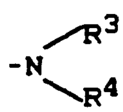 ,

worin
$R^3$ und $R^4$ die oben angegebene Bedeutung haben

oder

- für geradkettiges, verzweigtes oder cyclisches Alkyl oder Alkenyl mit bis zu 10 Kohlenstoffatomen steht, das substituiert sein kann durch Halogen, Aryl mit 6 bis 12 Kohlenstoffatomen, Furyl, Thienyl, Pyridyl, Pyrimidyl, Pyrazinyl, Pyridazinyl, Chinolyl, Isochinolyl, Hydroxy, Alkoxy, Alkylthio mit jeweils bis zu 6 Kohlenstoffatomen, Carboxy, Alkoxycarbonyl mit bis zu 6 Kohlenstoffatomen, Sulfo, Alkylsulfonyl mit bis zu 6 Kohlenstoffatomen, Phenylsulfonyl, Tolysulfonyl oder durch eine Gruppe der Formel

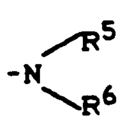 ,

worin

$R^5$ und $R^6$ gleich oder verschieden sind und Wasserstoff, Alkyl mit bis zu 8 Kohlenstoffatomen, Aryl mit 6 bis 12 Kohlenstoffatomen, Aralkyl mit 7 bis 14 Kohlenstoffatomen, Acyl mit bis zu 7 Kohlenstoffatomen, Alkylsulfonyl mit bis zu 6 Kohlenstoffatomen, Phenylsulfonyl oder Tolylsulfonyl bedeuten,

oder worin

$R^5$ und $R^6$ gemeinsam mit dem Stickstoffatom einen Ring aus der Reihe Pyrrolidino, Piperidino, Piperazino, Morpholino oder Thiomorpholino bilden und wobei dieser Ring substituiert sein kann durch Alkyl mit bis zu 4 Kohlenstoffatomen oder durch Phenyl

und

$R^2$ - für geradkettiges, verzweigtes oder cyclisches Alkyl mit bis zu 8 Kohlenstoffatomen steht,

in welchen das Ringkohlenstoffatom 5-Stellung S-Konfiguration hat und in welchen der Substituent $R^1$ in 4-Stellung zum Substituenten $COOR^2$ in 5-Stelllung cis konfiguriert ist,
gefunden, das durch gekennzeichnet ist,
daß man

Diohydropyrazine der allgemeinen Formel (II)

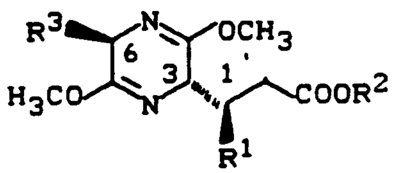

(II)

in welcher

$R^1$ und $R^2$ die angegebene Bedeutung haben,

und

$R^3$ - für geradkettiges oder verzweigtes Alkyl mit bis zu 4 Kohlenstoffatomen steht,

und in welchem das Kohlenstoffatom 6 des Dihydropyrazinrings in (II) R-konfiguriert ist, das Kohlenstoffatom 3 des Dihydropyrazinrings in (II) S-konfiguriert ist und das Kohlenstoffatom 1' in der Seitenkette S-konfiguriert ist, wenn $R^1$ die höhere Priorität hat als die Gruppe $CH_2COOR^2$ oder R-konfiguriert ist, wenn $R^1$ eine niedrigere Priorität hat als die Gruppe $CH_2COOR^2$
zunächst in inerten Lösemitteln mit Säuren hydrolysiert, dann aus den entstehenden sauren Aminosäuresalzen mit Basen in inerten Lösemitteln die freien Aminosäuren herstellt und diese anschließend cyclisiert.

Verwendet man als Ausgangsstoff (3S, 6R, 1′S)-2,5-Dimethoxy-6-isopropyl-3-(2′-methoxycarbonyl-1′-phenyl)ethyl-3,6-dihydro-1,4-pyrazin, so läßt sich das Verfahren durch folgendes Schema verdeutlichen:

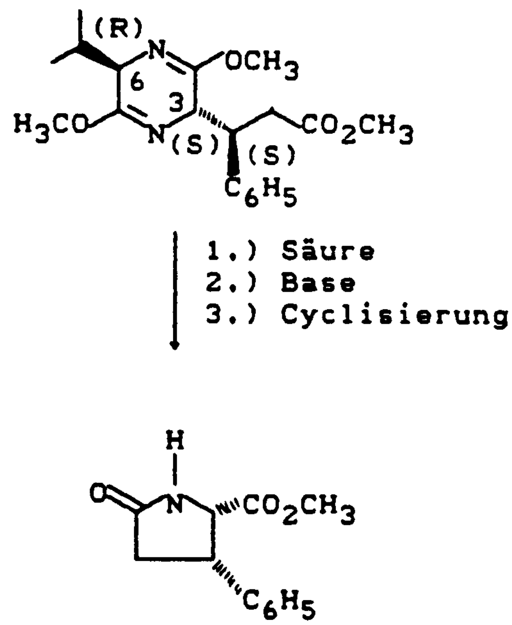


Als Lösemittel eignen sich die üblichen inerten Lösemittel, die sich unter den Reaktionsbedingungen nicht verändern. Hierzu gehören bevorzugt Wasser oder Alkohole wie beispielsweise Methanol, Ethanol, Propanol oder Isopropanol, oder Ether wie beispielsweise Diethylether, Dioxan oder Tetrahydrofuran, oder Chlorkohlenwasserstoffe wie beispielsweise Methylenchlorid, Chloroform oder Tetrachlorkohlenstoff, oder aber Gemische der genannten Lösemittel.

Als Säuren für die Hydrolyse eignen sich anorganische Säuren wie beispielsweise Chlorwasserstoffsäure, Bromwasserstoffsäure, Schwefelsäure oder Phosphorsäure, oder organische Carbon- oder Sulfonsäuren wie beispielsweise Methan-, Ethan-, Toluol- oder Benzolsulfonsäure oder Essigsäure oder Propionsäure.

Besonders bevorzugt wird die Hydrolyse in wäßrigen oder alkoholischen Lösungen mit Chlorwasserstoff als Säure durchgeführt.

Die Hydrolyse wird im allgemeinen bei einer Temperatur von 0°C bis +100°C, bevorzugt von +20°C bis +60°C durchgeführt.

Im allgemeinen arbeitet man bei Normaldruck. Das Verfahren kann aber ebenso bei erhöhtem oder erniedrigtem Druck durchgeführt werden.

Die bei der Hydrolyse anfallenden Glutaminsäuresalze können isoliert werden. Es hat sich jedoch hierbei als vorteilhaft erwiesen, die Aminosäuresalze ohne Reinigung direkt weiter zu verarbeiten.

Zur Freisetzung der Aminosäure werden die Salze in einem inerten Lösemittel mit Basen behandelt.

Als Lösemittel eignen sich hierbei die üblichen inerten Lösemittel, die sich unter den Reaktionsbedingungen nicht verändern. Hierzu gehören bevorzugt Wasser oder Alkohole wie beispielsweise Methanol, Ethanol, Propanol oder Isopropanol, oder Ether wie Diethylether, Dioxan oder Tetrahydrofuran, Acetonitril, Dimethylformamid oder Hexamethylphosphorsäuretriamid oder aber Gemische der genannten Lösemittel.

Als Basen eignen sich die üblichen basischen Verbindungen. Hierzu gehören bevorzugt anorganische Basen wie Alkali- oder Erdalkalihydroxid beispielsweise Natriumhydroxid, Kaliumhydroxid, Calciumhydroxid oder Bariumhydroxid, oder Alkalicarbonate wie Natriumcarbonat, Natriumhydrogencarbonat oder Kaliumcarbonat, oder Ammoniak oder von Ammoniak abgeleitete organische Amine wie Di-oder Trialkylamine beispielsweise Triethylamin oder Diisopropylamin, oder andere tertiäre Amine wie beispielsweise Pyridin, Dimethylaminopyridin, Picolin oder Lutidin.

Bevorzugt arbeitet man in wäßrigen oder alkoholischen Lösungen mit Ammoniak oder Triethylamin als Basen.

Die Behandlung der Aminosäuresalze mit Basen erfolgt im allgemeinen in einem Temperaturbereich von 0°C bis +100°C, bevorzugt von +20°C bis +40°C.

Im allgemeinen arbeitet man bei Normaldruck. Ebenso ist es möglich,das Verfahren bei erhöhtem oder erniedrigtem Druck durchzuführen.

Die freien Aminosäuren können isoliert werden. Im allgemeinen hat es sich aber als günstig erwiesen, ohne Isolierung der Aminosäure direkt weiter zu arbeiten.

Die Cyclisierung der freien Aminosäure wird im allgemeinen ohne Lösemittel in einem Temperaturbereich von +50°C bis +200°C, bevorzugt von +70°C bis +120°C durchgeführt.

Die Cyclisierung kann bei Normaldruck, bei erhöhtem oder bei erniedrigtem Druck durchgeführt werden. Im allgemeinen arbeitet man bei Normaldruck.

Das erfindungsgemäße Verfahren kann beispielsweise durchgeführt werden, indem man die Dihydropyrazine mit wäßriger Salzsäure behandelt, dann die anfallenden Glutaminsäuresalze isoliert und ohne Reinigung mit wäßrigem Ammoniak behandelt, dann die freien Aminosäuren isoliert und ohne Lösemittel, beispielsweise im Kugelohr, erhitzt.

Die als Ausgangsstoffe eingesetzten Dihydropyrazin der allgemeinen Formel (II)

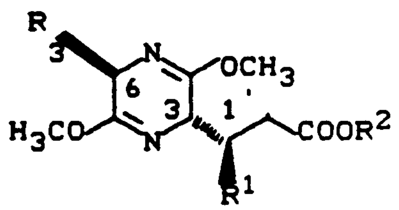

(II)

in welcher

$R^1$ - für Aryl mit 6 bis 14 Kohlenstoffatomen steht, das bis zu 5-fach gleich oder verschieden substituiert sein kann durch Alkyl, Alkoxy, Alkylthio mit jeweils bis zu 8 Kohlenstoffatomen, durch Aryl, Aryloxy, Arylthio mit jeweils 6 bis 12 Kohlenstoffatomen, durch Aralkyl mit 7 bis 14 Kohlenstoffatomen, durch Halogen, Trifluormethyl, Trifluormethoxy, Difluormethoxy, Trifluormethylthio, Nitro, Cyano, Carboxy, Alkoxycarbonyl mit bis zu 8 Kohlenstoffatomen, Sulfo, Phenylsulfonyl, Tolylsulfonyl, Alkylsulfonyl mit bis kzu 8 Kohlenstoffatomen, Hydroxy oder
durch eine Gruppe der Formel

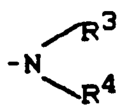

,

worin

$R^3$ und $R^4$ gleich oder verschieden sind und Wasserstoff, Alkyl mit bis zu 8 Kohlenstoffatomen, Aryl mit 6 bis 12 Kohlenstoffatomen, Aralkyl mit 7 bis 14 Kohlenstoffatomen, Acyl mit 2 bis 7 Kohlenstoffatomen, Alkylsulfonyl mit bis zu 6 Kohlenstoffatomen, Phenylsulfonyl oder Tolylsulfonyl bedeuten,

oder

- für einen heterocyclischen Ring der Reihe Furyl, Thienyl, Pyridyl, Pyrimidyl, Pyrazinyl, Pyridazinyl, Chinolyl oder Isochinolyl steht, wobei diese Ringe bis zu 3-fach gleich oder verschieden substituiert sein können durch Alkyl, Alkoxy, Alkylthio mit jeweils bis zu 6 Kohlenstoffatomen, Halogen, Phenyl, Nitro, Cyano oder
durch eine Gruppe der Formel

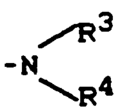

,

worin

$R^3$ und $R^4$ die oben angegebene Bedeutung haben,

oder

- für geradkettiges, verzweigtes oder cyclisches Alkyl oder Alkenyl mit bis zu 10 Kohlenstoffatomen steht, das substituiert sein kann durch Halogen, Aryl mit 6 bis 12 Kohlenstoffatomen, Furyl, Thienyl, Pyridyl, Pyrimidyl, Pyrazinyl, Pyridazinyl, Chinolyl, Isochinolyl, Hydroxy, Alkoxy, Alkylthio mit jeweils bis zu 6 Kohlenstoffatomen, Carboxy, Alkoxycarbonyl mit bis zu 6 Kohlenstoffatomen, Sulfo, Alkylsulfonyl mit bis zu 6 Kohlenstoffatomen, Phenylsulfonyl, Tolysulfonyl oder
durch eine Gruppe der Formel

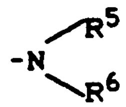 ,

worin

$R^5$ und $R^6$ gleich oder verschieden sind und Wasserstoff, Alkyl mit bis zu 8 Kohlenstoffatomen, Aryl mit 6 bis 12 Kohlenstoffatomen, Aralkyl mit 7 bis 14 Kohlenstoffatomen, Acyl mit bis zu 7 Kohlenstoffatomen, Alkylsulfonyl mit bis zu 6 Kohlenstoffatomen, Phenylsulfonyl oder Tolylsulfonyl bedeuten,

oder worin

$R^5$ und $R^6$ gemeinsam mit dem Stickstoffatom einen Ring aus der Reihe Pyrrolidino, Piperidino, Piperazino, Morpholino oder Thiomorpholino bilden und wobei dieser Ring substituiert sein kann durch Alkyl mit bis zu 4 Kohlenstoffatomen oder durch Phenyl,

$R^2$ - für geradkettiges, verzweigtes oder cyclisches Alkyl mit bis zu 8 Kohlenstoffatomen steht,

und

$R^3$ - für geradkettiges oder verzweigtes Alkyl mit bis zu 4 Kohlenstoffatomen steht,

und worin das Kohlenstoffatom 3 des Dihydropyrazinrings S-konfiguriert und das Kohlenstoffatom 6 R-konfiguriert ist, das Kohlenstoffatom 1′ in der Seitenkette S-konfiguriert ist, wenn $R^1$ die höhere Priorität hat als die Gruppe $CH_2COOR^2$ oder R-konfiguriert ist, wenn $R^1$ eine niedrigere Priorität hat als die Gruppe $CH_2COOR^2$,
sind neu und können hergestellt werden, indem man
Verbindungen der allgemeinen Formel (III)

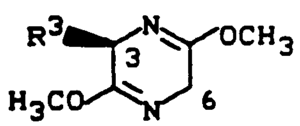 (III),

in welcher
$R^3$ die oben angegebene Bedeutung hat und das Kohlenstoffatom 3 des Dihydropyrazinrings R-konfiguriert ist, zunächst mit einer stark basischen metallorganischen Verbindung zu den in 6-Stellung durch das Metall der metallorganischen Verbindungen monosubstituierten Derivaten von (III) umsetzt,
dann diese Metallderivate mit einem cis-substituierten
Acrylester der allgemeinen Formel (IV)

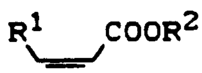 (IV),

in welcher $R^1$ und $R^2$ die oben angegebene Bedeutung haben, in inerten Lösemitteln umsetzt und anschließend mit einer Säure neutralisiert.

Aus DOS 29.34.252 ist bekannt, daß man durch Alkylieren oder Umsetzen mit Carbonylverbindungen aus Lactimethern vom Typ III enantiomerenreine 2-Aminosäuren oder Serine erhält.

Außerdem ist bekannt [Chem. Scripta 25, 105 (1985)], daß Lactimether vom Typ (III) mit trans-substituierten α,β-ungesättigten Carbonsäureestern 2,3-threo-Aminosäuren und daraus 4,5-trans-substituierte γ-Butyrolactame liefern.

Aufgrund der Kenntnis des Standes der Technik war nicht zu erwarten, daß cis-substituierte α,β-ungesättigte Carbonsäureester vom Typ (IV) mit Lactimethern vom Typ (III) selektiv zu den enantiomerenreinen Dihydropyrazinen des Typs (II) reagieren, die wiederum Vorstufen zu enantiomerenreinen cis-konfigurierten γ-Butyrolactamen der Formel (I) darstellen.

Verwendet man als Ausgangsstoffe (3R)-2,5-Dimethoxy-3-isopropyl-3,6-dihydro-1,4-pyrazin und cis-Zimtsäuremethylester so läßt sich das Verfahren durch folgendes Schema verdeutlichen:

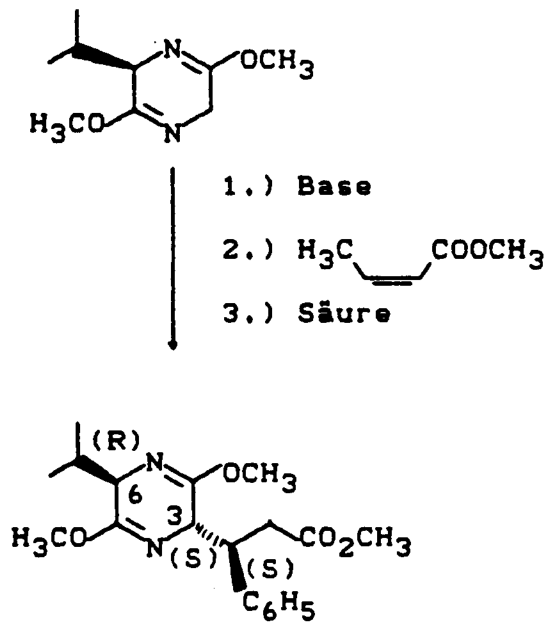


Die als Ausgangsstoffe eingesetzten Verbindungen der allgemeinen Formel (III) sind bekannt (DOS 29.34.252).

Die als Ausgangsstoffe eingesetzten Acrylester der allgemeinen Formel (IV) sind bekannt oder können nach bekannten Methoden hergestellt werden [vgl. z.B. Houben-Weyl, "Methoden der organischen Chemie", 4. Auflage, Band 5/1b, S. 728 ff.].

Als Lösemittel eignen sich die üblichen inerten organischen Lösemittel, die sich bei den Reaktionbedingungen nicht verändern. Hierzu gehören bevorzugt Ether wie Diethylether, Tetrahydrofuran, Dioxan, Glykolmono- oder Dimethylether, oder Amide wie Dimethylformamid, Hexamethylphosphorsäuretriamid, Dimethylacetamid, oder Dimethylsulfoxid. Ebenso ist es möglich, Gemische der genannten Lösemittel einzusetzen.

Als stark basische metallorganische Verbindungen eignen sich die üblichen alkalimetallorganischen Verbindungen. Hierzu gehören bevorzugt Alkalialkoholate wie Natriummethylat, Kaliummethylat, Natriumethylat, Kaliumethylat oder Kalium-tert-butylat, oder lithiumorganische Verbindungen wie n-, iso-, oder tert-Butyllithium oder nyllithium, oder Alkaliamide wie beispielsweise Natriumamid, Lithiumdiisopropylamid, Lithiumtetramethylpiperidid oder Natrium-bis-(trimethylsilyl)amid.

Als Säuren zum Neutralisieren eignen sich die üblichen anorganischen oder organischen Säuren. Hierzu gehören bevorzugt anorganische Säuren wie Salzsäure, Bromwasserstoffsäure, Schwefelsäure oder Phosphorsäure oder organische Carbon- oder Sulfonsäuren wie Essigsäure, Propionsäure, Weinsäure oder Zitronensäure, Methan- oder Ethansulfonsäure.

Das Verfahren wird im allgemeinen in einem Temperaturbereich von -80°C bis 0°C, bevorzugt von -70°C bis -20°C durchgeführt.

Das Verfahren wird im allgemeinen bei Normaldruck durchgeführt. Ebenso ist es möglich bei erhöhtem oder erniedrigtem Druck zu arbeiten.

Verwendet man statt des an Kohlenstoffatom 3 R-konfigurierten 2,5-Dimethoxy-3-isopropyl-3,6-dihydro-1,4-pyrazins das entsprechende S-Enantiomere als Ausgangsmaterial, so entstehen die Verbindungen II mit (6S), (3R), (1′R) Konfiguration.

Die erfindungsgemäßen enantiomerenreine 4,5-disubstituierte γ-Butyrolactame der Formel (I) sind wertvolle Zwischenprodukte für pharmazeutische Wirkstoffe.

Es ist beispielsweise aus EP 172 514 bekannt, daß Clausenamid [(±) (3S*), (4R*), (5R*), (7S*)-3-Hydroxy-α-hydroxybenzyl-1-methyl-4-phenyl-pyrrolidin-2-on] welches aus dem wäßrigen Extrakt von Clausena lansium (lour) Skeels isoliert wird, ein Racemat ist. Mit Hilfe der erfindungsgemäßen enantiomerenreinen 4,5-disubstituierten γ-Butyrolactamen (I) ist es nun möglich (+) (3R), (4S), (5S), (7R)-3-Hydroxy-5-α-hydroxybenzyl-1-methyl-4-phenyl-pyrrolidin-2-on sowie Derivate in enantiomerenreiner Form zu synthetisieren.

Die Reduktion der Verbindung der Formel (IX) zu Verbindungen der Formel (X) in Schritt [C] erfolgt nach der gleichen Methode und unter den gleichen Bedingungen wie bereits für die Reduktion der Verbindungen (V) zu den Verbindungen (IIb) angegeben ist.

Die Oxidation von Verbindungen der Formel (X) zu Verbindungen der Formel (III) in Schritt [D] erfolgt nach den gleichen Methoden und unter den gleichen Bedingungen wie bereits für die Oxidation von Verbindungen der Formel (IIa) zu Verbindungen der Formel (V) angegeben ist,

Die Ausgangsverbindungen der Formel (VI) sind literaturbekannt oder können nach literaturbekannten Methoden hergestellt werden [G.H. Cocolas, W.H. Hartung, J. Am. Chem. Soc. 79, 5203 (1957); F. Zymalkowski, P. Pachaly, Chem. Ber. 100, 1137 (1967)].

Verwendet man Verbindungen der Formel I mit (4R), (5R)-Konfiguration, die sich aus Verbindungen II mit (6S), (3R), (1'R)-Konfiguration herstellen lassen, als Ausgangsmaterial, so erhält man (-) (3S), (4R), (5R), (7S)-3-Hydroxy-5-α-hydroxybenxyl-1-methyl-4-phenyl-pyrrolidin-2-on.

So kann beispielsweise die Synthese von (+) (3S), (4R), (5R), (7S)-3-Hydroxy-5-α-hydroxybenxyl-1-methyl-4-phenyl-pyrrolidin-2-on aus dem erfindungsgemäßen, wie beschrieben hergestellten (4S,5S)-5-Methoxycarbonyl-4-phenyl-pyrrolidin-2-on) gemäß folgendem Schema durchgeführt werden:

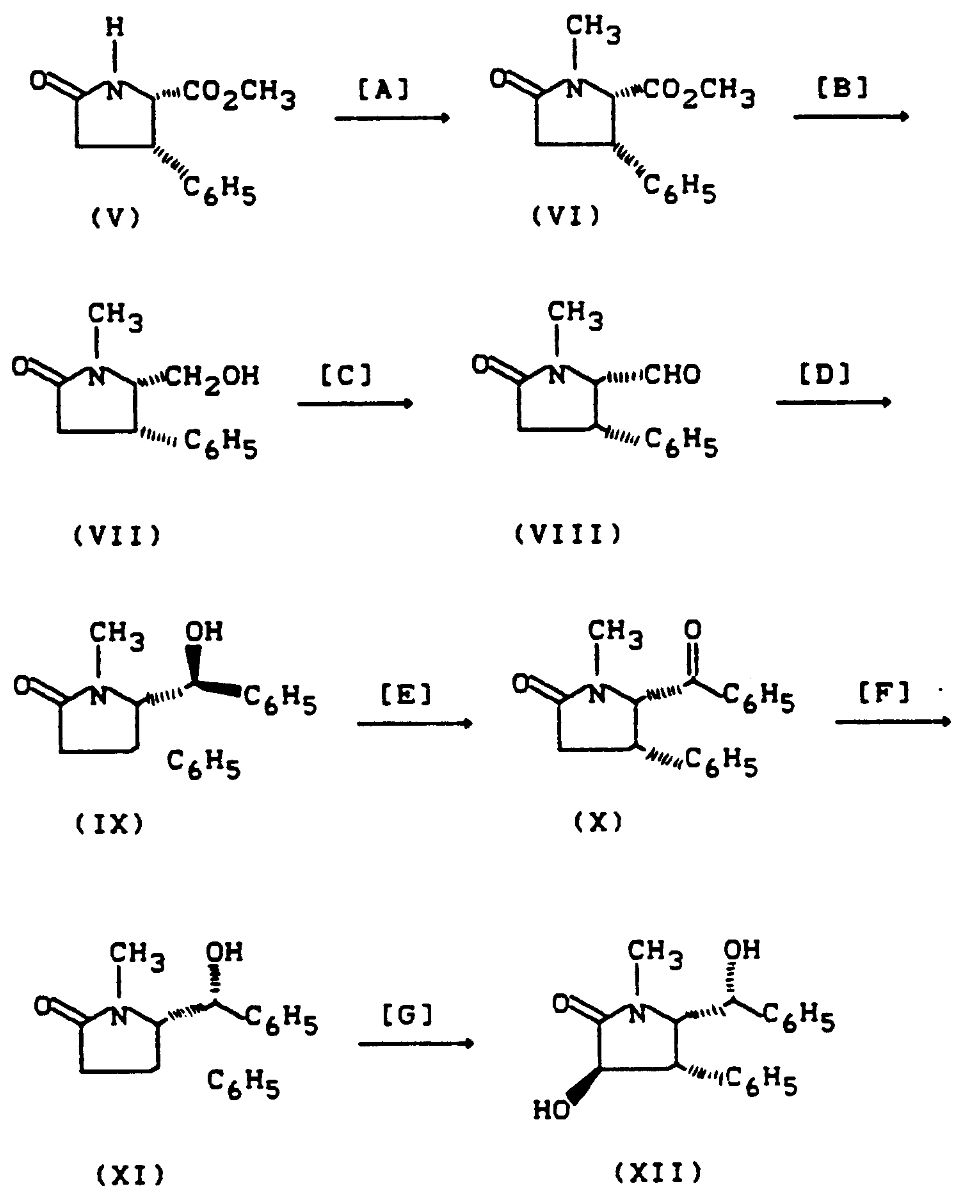


(V) (VI)

(VII) (VIII)

(IX) (X)

(XI) (XII)

Danach wird im Schritt [A] (4S,5S)-5-Methoxycarbonyl-4-phenyl-pyrrolidin-2-on (V) mit einem Methylierungsmittel wie beispielsweise Methylbromid, Methyliodid, Methyl-p-toluolsulfonat, Diazomethan oder Dimethylsulfat gegebenenfalls in Anwesenheit einer Base wie Natrium, Natriumhydrid, Natriumamid, Butyllithium oder Lithiumdiisopropylamid in geeigneten Lösungsmitteln, wie Diethylether, Tetrahydrofuran, Dimethylformamid, Hexamethylphosphorsäuretriamid oder Gemische der Lösungsmittel bei Temperaturen von -20°C bi +80°C, bevorzugt von 0°C bis +40°C methyliert. Besonders bevorzugt wird die Methylierung in Schritt [A] mit Methyliodid in einem Gemisch Tetrahydrofuran und Hexamethylphosphorsäuretriamid in Anwesenheit von Lithiumdiisopropylamid als Base durchgeführt.

Die Reduktion von (VI) zu (4S, 5S)-5-Hydroxymethyl-1-methyl-4-phenylpyrrolidin-2-on (VII) in Schritt [B] wird bevorzugt mit komplexen Metallhydriden wie beispielsweise Lithium-hydrido-triethyl-borat, Lithium-hydrido-tris(1-methylpropyl)borat oder Natriumborhydrid in inerten Lösungsmitteln wie Ethern beispielsweise Diethylether, Tetrahydrofuran oder Dioxan bei Temperaturen von -30°C bis +10°C, bevorzugt von -20°C bis 0°C durchgeführt.

Die Oxidation von (VII) zu (4S,5S)-5-Formyl-1-methyl-4-phenyl-pyrrolidin-2-on (VIII) in Schritt [C] wird mit Dimethylsulfoxid als Oxidationsmittel unter Zusatz von Trifluoracetanhydrid in Chlorkohlenwasserstoffen wie beispielsweise Dichlormethan oder Chloroform, oder in Ethern wie Diethylether, Dioxan oder Tetrahydrofuran bei Temperaturen von -80°C bis 0°C, bevorzugt von -60°C bis 0°C durchgeführt.

In Schritt [D] wird die Formylverbindung (VIII) in geeigneten Lösungsmitteln wie Ether beispielsweise Diethylether oder Tetrahydrofuran in einem Temperaturbereich von -20°C bis +50°C, bevorzugt von -10°C bis +30°C mit Phenylmagnesiumbromid zu (4S, 5S, 7S)-5-Hydroxymethylphenyl-1-methyl-4-phenylpyrrolidin-2-on (IX) umgesetzt.

Die Oxidation von (IX) zu (4S, 5S)-5-Benzoyl-1-methyl-4-phenylpyrrolidin-2-on (X) in Schritt [E] erfolgt unter den gleichen Bedingungen wie sie bereits für die Oxidation von (VII) zu (VIII) in Schritt [C] angegeben sind.

Die Reduktion von (X) zu (4S, 5S, 7R)-5-Hydroxymethylphenyl-1-methyl-4-phenylpyrrolidin-2-on (XI) in Schritt [F] erfolgt unter den gleichen Bedingungen wie sie bereits für die Reduktion von (VI) zu (VII) in Schritt [B] angegeben sind.

Die Hydroxylierung von (XI) zu (+)-(3R,4S,5S,7R)-3-Hydroxy-5-hydroxymethylphenyl-1-methyl-4-phenylpyrrolidin-2-on [(+)-Clausenamid] (XII) in Schritt [G] beschrieben, wird mit Oxidationsmittel wie Molybdänperoxidihyd-Pyridin oder Sauerstoff in Anwesenheit von Phosphiten wie Trialkylphosphite beispielsweise Trimethylphosphit, Triethylphosphit oder Tripropylphosphit, sowie in Anwesenheit von Basen, beispielsweise metallorganischen Basen wie Lithiumdiisopropylamid oder Butyllithium in inerten organischen Lösemitteln wie Ethern, beispielsweise Diethylether oder Tetrahydrofuran, oder Hexamethylphosphorsäuretriamid oder Gemische derselben in einem Temperaturbereich von -80°C bis 0°C durchgeführt.

Herstellungsbeispiele

Beispiel 1

(3S,6R,1′S)-2,5-Dimethoxy-6-isopropyl-3-[2′-Methoxycarbonyl-1′-phenyl)ethyl-3,6-dihydro-1,4-pyrazin

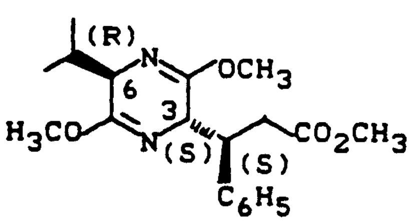

Die Lösung von 20 g (108,4 mmol) (3R)-2,5-Dimethoxy-3-isopropyl-3,6-dihydro-1,4-pyrazin in 120 ml abs. Tetrahydrofuran kühlte man auf -70°C und gab unter $N_2$-Atmosphäre 70 ml (108,4 mmol) einer 1,6 N Lösung von n-Butyllithium in Hexan zu. Man rührte 10 min bei dieser Temperatur und gab die Lösung von 19,36 g (119,2 mmol) cis-Zimtsäuremethylester in 60 ml abs. THF zu. Man rührte 12 h bei -70°C und 1 h bei -20°C, gab 6,83 ml (108,4 mmol) Eisessig, gelöst in 10 ml abs. THF zu, ließ auf Raumtemperatur kommen und goß die Reaktionsmischung auf 300 ml Eiswasser. Man extrahierte dreimal mit je 150 ml Essigester, trocknete die vereinigten organischen Extrakte über $MgSO_4$, filtrierte und zog das Solvens i. Vak. ab. Man erhielt 33.1 g (88% der Theorie) der rohen Titelverbindung als hellgelbes Öl. Flash-Chromatographie an Kieselgel (Amicon, Korngröße 20-45 μm; Eluens: Toluol / Essigester = 20/1) lieferte neben 4,9 g (13% der Theorie) des 6R,3S,1′R-Isomeren [$R_f$(Toluol : Essigester = 9:1) = 0,36] 27,4 g (73% der Theorie) der reinen Titelverbindung mit $R_f$ (Toluol : Essigester = 9:1) = 0,46 als schwach gelbes Öl.

$^1$H-NMR ($CDCl_3$, 250 MHZ): $\delta$ = 0.57 und 0,89 (je d, J=6,9 Hz, $\underline{(CH_3)}_2C$, 2H); 2,1 (m; $(CH_3)_2\underline{CH}$, 1H); ABM-Signal ($\delta_A$=2,91, $\delta_B$=3,18, $J_{AB}$=15,6 Hz, $J_{AM}$=$J_{BM}$=6,8 Hz, 2H, 2'-H); 3,08 (t, J=3,1 Hz, 1H, C(3)-H); 3,61, 3,65 und 3,72 (je s, je 3H, $OCH_3$); 3,9 (dt, J=6,8 Hz, J=3,1 Hz, 1H, C(1')-H); 4,34 (t, J=3,1 Hz, 1H, C(6)-H); 7,0 - 7,25 (m, 5H, Aromatan-H).

MS: $M^+$ 347

| | | | | |
|---|---|---|---|---|
| $C_{19}H_{26}N_2O_4$ (346.4) | Ber.: | C 65,9 | H 7,6 | N 8,1 |
| | ef.: | C 66,5 | H 7,7 | N 8,0 |

Beispiel 2

(4S,5S)-5-Methoxycarbonyl-4-phenylyrrolidin-2-on

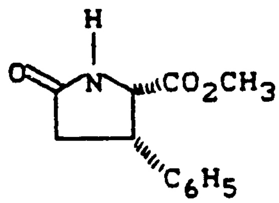

13,7 g (39,5 mmol) der Verbindung aus Beispiel 1 wurden in 317 ml 0,25 N Salzsäure 48 h kräftig gerührt. Man extrahierte dreimal mit je 100 ml Diethylether (Zurückgewinnung von Ausgangsmaterial). Dia wäßrige Lösung wurde lyophilisiert, der verbleibende Rückstand in 5 ml Wasser suspendiert und mit ca. 2,8 ml konz. Ammoniak auf pH=10 eingestellt. Man extrahierte fünfmal mit je 100 ml Essigsäureethylester unter Zusatz von NaCl bis zur Sättigung, trocknete über $MgSO_4$ und zog das Solvens i. Vak. ab. Das rohe Aminosäureestergemisch wurde am Kugelrohr 10 h bei 100°C/0,1 Torr gehalten. Man erhielt als Rückstand 5 g (58% dar Theorie) dar Titelverbindung mit $|\alpha|_D^{20}$ = 209,05 (c=0,54, MeOH); $R_f$ = 0,20 (Essigester)

$^1$H-NMR ($CDCl_3$, 200 mHz): $\delta$ = 2,78 (dd, J=7,5 Hz, J=2 Hz, 2H, C(3)-H); 3,30 (s, 3H, $OCH_3$); 3,99 (q, J=7,5 Hz, 1H, C(4)-H); 4,58 (d, J=7,5 Hz, 1H, C(5)-H); 6,85 (br, 1H, NH); 7,19 - 7,35 (m, 5H, $C_6H_5$).

| | | | | |
|---|---|---|---|---|
| ($C_{12}H_{13}NO_3$, 219,24) | Ber.: | C 65,7 | H 6,0 | N 6,4 |
| | Gef.: | C 65,5 | H 6,1 | N 6,4 |

Beispiel 3

(4S,5S)-N-Methyl-5-Methoxycarbonyl-4-phenylpyrrolidin-2-on

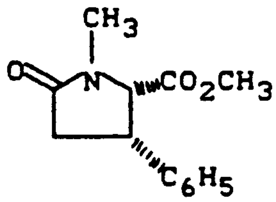

5 g (22,8 mmol) der Titelverbindung des Beispiels 2 wurden in 50 ml abs. Tetrahydrofuran und 15 ml abs. Hexamethylphosphorsäuretriamid im trockenen ausgeheizten Kolben unter $N_2$-Atmossphäre gelöst und auf -70°C gekühlt. Man tropfte bei dieser Temperatur die Lösung von 25,1 mmol Lithiumdiisopropylamid in THF/Hexan zu (hergestellt aus 15,7 ml 1,55 N BuLi in Hexan und 3,5 ml Diisopropylamin, in 15 ml THF), rührte 20 min bei dieser Temperatur nach und tropfte die Lösung von 4,2 ml (0,114 mol) Methyliodid in 5 ml abs. THF zu, ließ 1 h bei -70°C rühren und ließ innerhalb von 30 min. auf Raumtemperatur kommen. Sobald alles Startmaterial umgesetzt war (DC-Kontrolle) goß man die Reaktionsmischung auf 200 ml Phosphatpuffer (pH=7, pH-Kontrolle) und extrahierte viermal mit je 100 ml Essigester (zum Schluß unter Kochsalz-Zusatz). Trocknen ($MgSO_4$) und Einrotieren lieferte die rohe Titelverbindung, die über Kieselgel mit Essigester filtriert wurde. Man erhielt 5,05 g (94,6% der Theorie) der reinen Titelverbindung als farblosen Festkörper mit $R_f$ = 0,3 (Essigester) und $|\alpha|_D^{20}$ = 205,95 (c=0,38, MeOH) und Schmp.: 100°C

IR (KBr): $\delta$ = 1736, 1690 $cm^{-1}$

$^1$H-NMR (250 MHz, $CDCl_3$): $\delta$ = ABX-Signal ($\delta_a$ = 2,70, $\delta_B$ = 2,95, $J_{AB}$ = 17,5 Hz, $J_{AX}$ = 10 Hz, $J_{BX}$ = 11 Hz, 2H, C(3)-H); 2,89 (s, 3H, N-$CH_3$); 3,30 (s, 3H, $OCH_3$); 3,91 (q, J=10 Hz, 1H, C(4)-H); 4,39 (d, J=9-10 Hz, 1H, C(5)-H); 7,18 - 7,38 (m, 5H, $C_6H_5$).

| | | | | |
|---|---|---|---|---|
| $C_{13}H_{15}NO_3$ (233,27) | Ber.: | C 66,9 | H 6,5 | N 6,0 |
| | Gef.: | C 67,1 | H 6,5 | N 6,0 |

Beispiel 4

(4S,5S)-5-Hydroxymethyl-1-methyl-4-phenylpyrrolidin-2-on

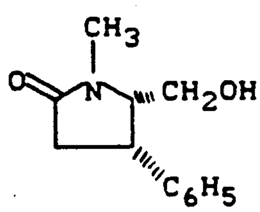

Zur Lösung von 3 g (12,8 mmol) der Titelverbindung aus Beispiel 3 in 33 ml abs. Tetrahydrofuran tropfte man bei -15 bis -20°C unter $N_2$-Atmosphäre 25,6 mmol Li $B(Et)_3H$ (als 1 M-Lösung in THF, 25,6 ml). Man ließ 1 h bei -20°C und 1 h bei 0°C nachrühren, goß die Raktionsmischung in ca. 200 ml eiskalte 2 N-Salzsäure, rührte 30 min. kräftig durch und extrahierte zweimal mit je 200 ml Essigester. Man sättigte die wäßrige Phase mit Kochsalz und extrahierte nochmals zweimal mit je 200 ml Essigester. Die gesammelten organischen Extrakte wurden mit wenig Wasser gewaschen, über

$MgSO_4$ getrocknet und einrotiert. Der Rückstand wurde mit wenig Ether zur Kristallisation gebracht und anschließend mit Pentan ausgefällt, bis an der Eintropfstelle keine Trübung mehr zu beobachten war. Nach Absaugen und Trocknen erhielt man 2,07 g (79% der Theorie) der Titelverbindung mit Schmp.: 93-95°C.

IR (KBr): $\nu$ = 3324, 1687 $cm^{-1}$.
$^1$H-NMR $CDCl_3$, 300 MHz): $\delta$ = AB-Teil von ABM System, $\delta_A$ = 2,59, $\delta_B$ = 2,97 (je dd, $J_{AB}$ = 15 Hz, $J_{AM}$ = 7,5 Hz, $J_{BM}$ = 9 Hz, 2H, C(3)-H); 2,97 (s, 3H, N-$CH_3$); AB-Teil von ABM System, $\delta_A$ = 3,36, $\delta_B$ = 3,62 (je dd, $J_{AB}$ = 11,2 Hz, $J_{AM}$ = $J_{BM}$ = 3 hz, 2H, C(7)-H); 3,72 - 3,85 (m, 2H, C(4)-H, C(5)-H); 7,32 (m, 5H, $C_6H_5$).

| | | | | |
|---|---|---|---|---|
| $CV_{12}H_{15}NO_2$ (205,26) | Ber.: | C 70,2 | H 7,4 | N 6,8 |
| | Gef.: | C 70,0 | H 7,4 | N 6,8 |

Beispiel 5

(4S,5S)-5-Formyl-1-methyl-4-phenylpyrrolidin-2-on

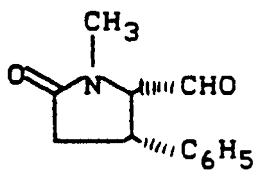

Zur Lösung von 1,9 ml (28 mmol) abs. Dimethylsulfoxid in 14 ml abs. Dichlormethan tropfte man unter $N_2$-Atmosphäre innerhalb 10 min. bei -60°C die Lösung von 2,97 ml Trifluoracetanhydrid in 5,6 ml abs. Dichlormethan. Man ließ 15 min. bei dieser Temperatur rühren und tropfte die Lösung von 2,9 g (14 mmol) der Titelverbindung aus Beispiel 4 in 25 ml Dichlormethan so zu, daß die Temperatur -60°C nicht überstieg. Man ließ 90 min. bei -60°C nachrühren, erwärmte kurz auf -30°C (5-10 min.) und kühlte wieder auf -60°C ab. Man gab langsam bei dieser Temperatur 5,6 ml abs. Triethylamin zu, ließ 30 min. bei -60°C rühren und erwärmte auf Raumtemperatur. Man gab 60 ml Wasser zu, trennte die Phasen und extrahierte die wäßrige Phase dreimal mit je 25 ml Dichlormethan. Die gesammelten organischen Extrakte wurden zweimal mit je 300 ml Wasser gewaschen, über Magnesiumsulfat getrocknet und extrahiert. Man erhielt 2,83 g (100% der Theorie) der Titelverbindung mit $R_f$ = 0,25 (Essigester) (nach $^1$H-NMR-Spektrum zu 91% rein). Das so erhaltene Rohprodukt wurde nach Trocknung (24 h, HV) direkt weiter umgesetzt.

IR ($CHCl_3$): $\nu$ = 1734, 1689 $cm^{-1}$
$^1$H-NMR (300 MHz, $CDCl_3$): $\delta$ = 2,79 (dd, J=5,3 Hz, J=9,7 Hz, 2H, C(3)-H); 2,91 (s, 3H, N-$CH_3$); 4,02 (q, J=9,7 Hz, 1H, C(4)-H); 4,30 (dd, J=1 Hz, J=9,7 Hz, 1H, C(5)-H); 7,3 (m, 5H, $C_6H_5$); 9,17 (d, J=1 Hz, 1H, CHO).

Beispiel 6

(4S,5S,7S)-5-α-Hydroxybenzyl-1-methyl-4-phenylpyrrolidin-2-on

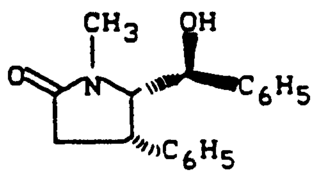

Zu 0,39 g Mg-Späne tropfte man unter $N_2$ die Lösung von 2,48 g (1,67 ml, 0,0156 mol) Brombenzol in 4,4 ml abs. Tetrahydrofuran so zu, daß das THF gelinde siedete. Man gab anschließend 10 ml abs. THF zu und erhitzte unter Rückfluß zum Sieden bis alles Magnesium gelöst war (1-2 h).
Man kühlte auf 0°C und tropfte unter kräftigem Rühren die Lösung von 2,47 g (0,012 mol) der Titelverbindung aus Beispiel 5 in 25 ml abs. THF so zu, daß die Temperatur 5°C nicht überstieg. Gegebenenfalls mußte abs. THF zur besseren Rührfähigkeit zugegeben werden. Man rührte anschließend 1 h bei 0 - 5°C und goß die Reaktionsmischung auf 35 ml 0,5 N HCl-Eis und extrahierte viermal mit je 30 ml Essigester und zweimal mit je 30 ml Dichlormethan. Die gesammelten Essigester- und Dichlormethan-Extrakte wurden (getrennt !) zweimal mit je 20 ml Wasser gewaschen, vereinigt und über Magnesiumsulfat getrocknet. Der nach Abziehen des Solvens (i.Vak.) verbleibende Rückstand wurde mit 10 ml Ether bis zur Kristallisation verrieben. Anschließend gab man 50 ml Pentan langsam zu und ließ über Nacht im Kühlschrank stehen. Absaugen des Festkörpers ergab 2,5 g (74,3% der Theorie) der Titelverbindung mit
Schmp.: 210-212°C
$|\alpha|_D^{20}$ = 173.1 (c=0,5, MeOH)

IR (KBr): $\nu$ = 3362 (br), 1654 $cm^{-1}$
$^1$H-NMR (300 MHz, $d_6$-DMSO): $\delta$ = 2,21 (s, 3H, $NCH_3$); 2,24 (dd, A-Teil vom ABM-System, $J_{AB}$=15,7 Hz, $J_{AM}$=9,4 Hz, 1H, cis-C(3)-H); 3,05 (dd, B-Teil vom ABM-System, $J_{BM}$=12,7 Hz, 1H, trans-C(3)-H); 3,80 (dt, M-Teil vom ABM-System, $J_{AM}$=9,4 Hz, $J_B$=12,7 Hz, $J_{4,5}$=8,5 Hz, 1H, C(4)-H); 4,15 (dd, J=8,5 hz, J=1 Hz, 1H, C(5)-H); 4,26 (dd, J=6 Hz, J=1 Hz, 1H, C(7)-H); 5,35 (d, J=6 Hz, 1H, OH); 7,15 - 7,5 (m, 10H, $C_6H_5$).

| | | | |
|---|---|---|---|
| $C_{18}H_{19}NO_2$ (281,4) | Ber.: | C 76,8 | H 6,8 |
| | Gef.: | C 76,5 | H 6,8 |

Beispiel 7

(4S,5S)-5-Benzoyl-1-methyl-4-phenylpyrrolidin-2-on

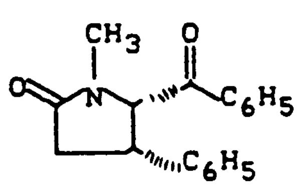

Zur Lösung von 1,2 ml (0,0171 mol) abs. Dimethylsulfoxid in 8,7 ml abs. Dichlormethan tropfte man unter $N_2$-Atmosphäre innerhalb 10 min. bei -60°C die Lösung von 1,8 ml Trifluoracetanhydrid in 34 ml abs. Dichlormethan. Man ließ 15 min. bei dieser Temperatur nachrühren und tropfte die Lösung von 2,4 g (0,0085 mol) der Titelverbindung aus Beispiel 6 in ca. 70 ml abs. Dichlormethan so zu, daß die Temperatur -60°C nicht überstieg. Man ließ 90 min. bei -60°C

nachrühren, erwärmte kurz auf -30°C (9-10 min.) und kühlte wieder auf -60°C ab. Man gab langsam bei dieser Temperatur 3,4 ml Triethylamin zu, ließ 20 min. bei -60°C rühren und erwärmte auf Raumtemperatur. Man gab 37 ml Wasser zu, trennte die Phasen und extrahierte die wäßrige Phase dreimal mit je 25 ml Dichlormethan. Die vereinigten organischen Extrakte wurden zweimal mit je 30 ml Wasser gewaschen, über Magnesiumsulfat getrocknet und einrotiert. Der Rückstand wurde zweimal mit je 20 ml Ether abrotiert. Man erhielt 2,3 g (100% der Theorie) der Titelverbindung als Festkörper mit Schmp.: 115-116°C und $R_f$ = 0,25 (Essigester). Das Rohprodukt, das nach $^1$H-NMR-Spektrum rein war, wurde direkt weiter umgesetzt.

IR (KBr): $\nu$ = 1695, 1682 $cm^{-1}$

$^1$H-NMR (300 MHz, $CDCl_3$): $\delta$ = 2,78 und 2,91 (AB-Teil vom ABM-Spektrkum, $J_{AB}$=16,5 Hz, $J_{AM}$=$J_{BM}$=8,3 Hz, 2H, C(3)-H); 2,88 (s, 3H, N-$CH_3$); 4,02 (q, J=8,3 Hz, 1H, C(4)-H); 5,42 (d, J=8,3 Hz, 1H, C(5)-H); 7,0, 7,21, 7,59, 7,50 (je m, 10H, $C_6H_5$).

Beispiel 8

(4S,5S,7R)-5-α-Hydroxybenzyl-1-methyl-4-phenylpyrrolidin-2-on

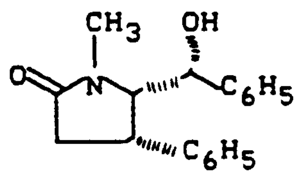

Zur Lösung von 2,3 g (8,2 mmol) der Titelverbindung aus Beispiel 7 in 20 bis 27 ml abs. Tetrahydrofuran tropfte man bei -15°C bis -20°C unter $N_2$-Atmophäre 8,3 mmol Li $B(Et)_3J$ (8,3 ml einer 1 M-Lösung in THF). Man ließ 1 h bei 0°C nachrühren, goß die Reaktionsmischung in 10 ml eiskalte 1 N HCl und extrahierte zweimal mit je 20 ml Essigester. Man sättigte die wäßrige Phase mit Kochsalz und extrahierte nochmals zweimal mit je 20 ml Essigester. Die vereinigten organischen Extrakte wurden über $MgSO_4$ getrocknet und einrotiert. Den Rückstand löste man in Dichlormethan und wusch zweimal mit je 10 ml Wasser. Die oragnische Phase wurde getrocknet ($MgSO_4$) und einrotiert. Der Rückstand wurde mit 10 ml Ether zur Kristallisation gebracht und anschließend langsam unter Rühren mit Pentan versetzt bis an der Eintropfstelle keine Trübung mehr zu beobachten war. Der Niederschlag wurde abgesaugt und getrocknet. Man erhielt 1,6 g (72% der Theorie) der Titelverbindung mit Schmp.: 189-195°C. Das Produkt ist nach $^1$H-NMR zu 95% rein und wurde direkt weiter umgesetzt.
Zur Analyse wurde aus Aceton umkristallisiert (Schmp.: 197-8°C).

IR (KBr): $\nu$ = 3251, 1692 $cm^{-1}$.

$^1$H-NMR (300 MHz, DMSO): $\delta$ = 1,97 und 2,05 (ABM-Signal, J $J_{AB}$=13,5 Hz, $J_{AM}$=8,2 Hz, $J_{BM}$=13 Hz, 2H, C(3)-H); 2,91 (s, 3H, N-$CH_3$); 3,82 (dt, $J_{AM}$=$J_{4,5}$=8,2 Hz, $J_{BM}$=13 Hz, 1H, C(4)-H); 4,27 (dd, J=8,2 Hz, J=1,5 Hz, 1H, C(5)-H); 4,65 (dd, J=1,5 Hz, J=3,5 Hz, 1H, C(7)-H); 5,34 (d, J=3,5 Hz, 1H, C-(7)- OH); 6,70, 7,11, 7,25 (je m, 10H, $C_6H_5$).

| | | | | |
|---|---|---|---|---|
| $C_{18}H_{19}NO_2$ (281,4) | Ber.: | C 76,8 | H 6,8 | N 5,0 |
| | Gef.: | C 77,0 | H 6,9 | N 5,0 |

Beispiel 9

(3R,4S,5S,7R)-3-Hydroxy-5-hydroxymethylphenyl-1-methyl-4-phenylpyrrolidin-2-on [(+)-Clausenamid]

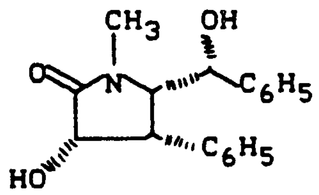

In einem i.Vak. ausgeheizten und mit Reinststickstoff belüfteten Kolben gab man die Lösung von 0,44 g (1,57 mmol) der Titelverbindung aus Beispiel 8 in 12,25 ml abs. Tetrahydrofuran und 3,25 ml abs. Hexamethylphosphorsäuretriamid und kühlte auf -70°C. Bei dieser Temperatur tropfte man die Lösung von 0,0038 mol Lithiumdiisopropylamid in 4,5 ml abs. THF/Hexan zu (hergestellt aus 0,553 ml Diisopropylamin in 2ml THF durch Zugabe von 2,6 ml einer 1,5 N-Lösung von n-Butyllithium in Hexan bei -20 °C bis 0 °C). Man rührte 1 h bei -70 °C bis -60 °C nach, gab 0,13 ml frisch dest. Trimethylphosphit (gelöst in wenig abs. Tetrahydrofuran) zu und leitete (über $H_2SO_4$ und $P_4O_{10}$ getrockneten) abs. Sauerstoff ein (50 - 100 ml/min). Sobald nach DC-Kontrolle ($SiO_2$; EE/MeOH : 2/1; $R_f$=0,3 für Titelverbindung, $R_f$=0,37 für Ausgangsmaterial, Anfärben mit Molybdatophosphorsäure Sprühreagenz der Fa. Merck, Darmstadt) sich das Verhältnis (Produkt/Ausgangsmaterial) nicht mehr änderte (2-3 h) goß man unter Eiskühlung auf 15 ml 0,5 N HCl und säuerte ggfs. auf pH 3 bis 4 an.

Man trennte die Phasen und extrahierte die wäßrige Phase viermal mit je 10 ml Essigester. Die vereinigten organischen Extrakte wurden dreimal mit je 10 ml Wasser gewaschen über $MgSO_4$ getrocknet und einrotiert. Man nahm den Rückstand in 5 - 10 ml Ether auf, rührte bis zu beginnenden Kristallisation und gab langsam unter Rühren soviel Pentan zu, bis in der Eintropfstelle keine Trübung mehr zu beobachten war. Man ließ über Nacht im Kühlschrank stehen und saugte ab. Man erhielt ca. 0,4 g eines rohen Festkörpers, der neben der Titelverbindung ca. 35 - 40% Ausgangsmaterial enthielt. Zur Reinigung wurde zweimal aus Methanol umkristallisiert. Man erhält dann die Titelverbindung in ca. 95% Reinheit. Verlustfreier und mit Rückgewinnung des reinen Ausgangsmaterials verläuft die Chromatographie über Aluminiumhydroxid (neutral). Hierzu wird das Rohprodukt auf Kieselgel aufgezogen (Lösen in MeOH in der Wärme, Zugabe von fünf Gewichtsteilen Kieselgel, Einrotieren und mehrfaches Abrotieren mit Essigester, bis ein staubtrockenes MeOH-freies Produkt resultiert). Das Adsorbat wird auf eine Säule mit $Al_2O_3$ (neutral, 50 Gewichtsteile) gegeben und mit Essigester zunächst das Ausgangsmaterial eluiert (flash-Chromatographie, Kontrolle mit DC und analyt. HPLC). Anschließend eluiert man die Titelverbindung mit Essigester/Methanol-Gemischen (40/1, 20/1, dann 10/1). Man kristallisierte mit Ether, rührte mit Wasser aus, saugte ab und erhielt nach Trocknung im HV (30-40°C, 24 h) 0,22 g (46,1% der Theorie) (+)-Clausenamid (Hydrat enthält 1/4 Mol $H_2O$) mit 152-3°C. (auth. (±)-Clausenamid; 236-7°C). Reinheit ca. 98% (nach $^1$H-NMR, enthält ca. 2% Ausgangsmaterial). 0,1 g das reinen Ausgangsmaterial konnten zurückgewonnen werden.

$|\alpha|_D^{20}$ = + 123,19 (C = 0,46, DMSO/$H_2O$ = 9/1 Vol-%)

IR (KBr): $\nu$ = 3402, 3321, 1689 $cm^{-1}$.

$^1$H-NMR (300 MHz, DMSO): $\delta$ = 3,01 (s, 3H, N-$CH_3$); 3,50 (dd, J=8 Hz, J=10,5 Hz, 1H, C(4)-H); 3,82 (dd, J=10 Hz, J=7 Hz, 1H, C(3)-H); 4,30 (dd, J=8 Hz, J=2 Hz, 1H, C(5)-H); 4,65 (dd, J=2 Hz, J=3 Hz, 1H, C(7)-H); 5,39 (d, J=7 Hz, 1H C(3)-OH); 5,45 (d, J=3 Hz, 1H, C(7)-OH); 6,61 - 6,64 (m, 2H, arom. H); 7,03 - 7,28 (m, 8H, arom. H).

| | | | |
|---|---|---|---|
| $C_{18}H_{19}NO_3$ + 1/4 $H_2O$ (315,37) | Ber.: | C 71,6 | H 6,5 |
| | Gef.: | C 71,6 | H 6,4 |

## Patentansprüche

**Patentansprüche für folgende Vertragsstaaten : AT, BE, CH, DE, FR, GB, IT, LI, NL, SE**

**1.** Enantiomerenreine 4,5-disubstituierte γ-Butyrolactame der allgemeinen Formel (I)

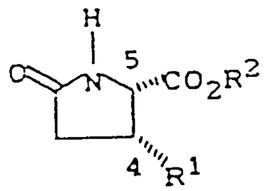

(I),

in welcher

$R^1$ - für Aryl mit 6 bis 14 Kohlenstoffatomen steht, das bis zu 5-fach gleich oder verschieden substituiert sein kann durch Alkyl, Alkoxy, Alkylthio mit jeweils bis zu 8 Kohlenstoffatomen, durch Aryl, Aryloxy, Arylthio mit jeweils 6 bis 12 Kohlenstoffatomen, durch Aralkyl mit 7 bis 14 Kohlenstoffatomen durch Halogen, Trifluormethyl, Trifluormethoxy, Difluormethoxy, Trifluormethylthio, Nitro, Cyano, Carboxy, Alkoxycarbonyl mit bis zu 8 Kohlenstoffatomen, Sulfo, Phenylsulfonyl, Tolylsulfonyl, Alkylsulfonyl mit bis zu 8 Kohlenstoffatomen, Hydroxy oder durch eine Gruppe der Formel

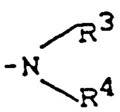

,

worin

$R^3$ und $R^4$ gleich oder verschieden sind und Wasserstoff, Alkyl mit bis zu 8 Kohlenstoffatomen, Aryl mit 6 bis 12 Kohlenstoffatomen, Aralkyl mit 7 bis 14 Kohlenstoffatomen, Acyl mit bis zu 7 Kohlenstoffatomen, Alkylsulfonyl mit bis zu 6 Kohlenstoffatomen, Phenylsulfonyl oder Tolylsulfonyl bedeuten,

oder

- für einen heterocyclischen Ring der Reihe Furyl, Thienyl, Pyridyl, Pyrimidyl, Pyrazinyl, Pyridazinyl, Chinolyl oder Isochinolyl steht, wobei diese Ringe bis zu 3-fach gleich oder verschieden substituiert sein können durch Alkyl, Alkoxy, Alkylthio mit jeweils bis zu 6 Kohlenstoffatomen, Halogen, Phenyl, Nitro, Cyano oder durch eine Gruppe der Formel

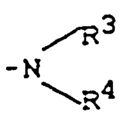

,

worin
$R^3$ und $R^4$ die oben angegebene Bedeutung haben,

oder

- für geradkettiges, verzweigtes oder cyclisches Alkyl oder Alkenyl mit bis zu 10 Kohlenstoffatomen steht, das substituiert sein kann durch Halogen, Aryl mit 6 bis 14 Kohlenstoffatomen, Furyl, Thienyl, Pyridyl, Pyrimidyl, Pyrazinyl, Pyridazinyl, Chinolyl, Isochinolyl, Hydroxy, Alkoxy, Alkylthio mit jeweils bis zu 6 Kohlenstoffatomen, Carboxy, Alkoxycarbonyl mit bis zu 6 Kohlenstoffatomen, Sulfo, Alkylsulfonyl mit bis zu 6 Kohlenstoffatomen, Phenylsulfonyl, Tolylsulfonyl oder
durch eine Gruppe der Formel

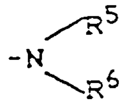,

worin

$R^5$ und $R^6$ gleich oder verschieden sind und Wasserstoff, Alkyl mit bis zu 8 Kohlenstoffatomen, Aryl mit 6 bis 12 Kohlenstoffatomen, Aralkyl mit 7 bis 14 Kohlenstoffatomen, Acyl mit bis zu 7 Kohlenstoffatomen, Alkylsulfonyl mit bis zu 6 Kohlenstoffatomen, Phenylsulfonyl oder Tolylsulfonyl bedeuten,

oder worin

$R^5$ und $R^6$ gemeinsam mit dem Stickstoffatom einen Ring aus der Reihe Pyrrolidino, Piperidino, Piperazino, Morpholino oder Thiomorpholino bilden und wobei dieser Ring substituiert sein kann durch Alkyl mit bis zu 4 Kohlenstoffatomen oder durch Phenyl

und

$R^2$ - für geradkettiges, verzweigtes oder cyclisches Alkyl mit bis zu 8 Kohlenstoffatomen steht,

in welchen das Ringkohlenstoffatom in 5-Stellung S-Konfiguration hat und in welchen der Substituent $R^1$ in 4-Stellung zum Substituenten $COOR^2$ in 5-Stellung cis konfiguriert ist.

**2.** Verbindungen der allgemeinen Formel (I) in Anspruch 1, in welchen

$R^1$ - für Phenyl oder Naphthyl steht, das bis zu 3-fach gleich oder verschieden substituiert sein kann durch Alkyl, Alkoxy mit jeweils bis zu 6 Kohlenstoffatomen, Methylthio, Phenyl, Phenoxy, Benzyl, Fluor, Chlor, Brom, Iod, Trifluormethyl, Trifluormethoxy, Difluormethoxy, Trifluormethylthio, Nitro, Cyano, Alkoxycarbonyl mit bis zu 6 Kohlenstoffatomen, Phenylsulfonyl, Tolylsulfonyl, Alkylsulfonyl mit bis zu 6 Kohlenstoffatomen, Hydroxy oder
durch eine Gruppe der Formel

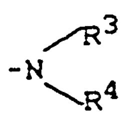,

in welcher

$R^3$ und $R^4$ gleich oder verschieden sind und Wasserstoff, Alkyl mit bis zu 6 Kohlenstoffatomen, Phenyl, Benzyl, Acetyl, Ethylcarbonyl, Benzoyl, Alkylsulfonyl mit bis zu 4 Kohlenstoffatomen, Tolylsulfonyl oder Phenylsulfonyl bedeuten,

oder

- für einen heterocyclischen Ring der Reihe Furyl, Thienyl, Pyridyl, Pyrimidyl, Chinolyl oder Isochinolyl steht, wobei diese Ringe substituiert sein Können durch Alkyl oder Alkoxy mit bis zu 4 Kohlenstoffatomen, durch Fluor, Chlor, Brom, Nitro, Cyano oder
durch eine Gruppe der Formel

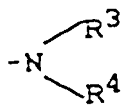 ,

worin
$R^3$ und $R^4$ die oben angegebene Bedeutung haben,

oder

- für geradkettiges, verzweigtes oder cyclisches Alkyl mit bis zu 8 Kohlenstoffatomen steht, das substituiert sein kann durch Fluor, Chlor, Brom, Phenyl, Furyl, Thienyl, Pyridyl, Pyrimidyl, Chinolyl, Isochinolyl, Hydroxy, Alkoxy oder Alkylthio mit bis zu 4 Kohlenstoffatomen, Alkoxycarbonyl mit bis zu 4 Kohlenstoffatomen, Alkylsulfonyl mit bis zu 4 Kohlenstoffatomen, Phenylsulfonyl, Tolylsulfonyl oder durch eine Gruppe der Formel

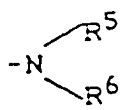 ,

worin

$R^5$ und $R^6$ gleich oder verschieden sind und Wasserstoff, Alkyl mit bis zu 6 Kohlenstoffatomen, Phenyl, Benzyl, Acetyl, Ethylcarbonyl, Benzoyl Alkylsulfonyl mit bis zu 4 Kohlenstoffatomen, Tolylsulfonyl oder Phenylsulfonyl bedeuten

oder in welcher

$R^5$ und $R^6$ gemeinsam mit dem Stickstoffatom einen Ring aus der Reihe Pyrrolidino, Piperidino, N-Methyl- oder N-Phenylpiperazino oder Morpholino bilden

und

$R^2$ - für geradkettiges oder verzweigtes Alkyl mit bis zu 6 Kohlenstoffatomen steht,

und in welchen das Ringkohlenstoffatom in 5-Stellung S-Konfiguration hat und in welchen der Substituent $R^1$ in 4-Stellung zum Substituenten $COOR^2$ in 5-Stellung cis konfiguriert ist.

**3.** Verbindungen der allgemeinen Formel (I) in Anspruch 1, in welcher

$R^1$ - für Phenyl steht, das substituiert sein kann durch Alkyl oder Alkoxy mit bis zu 4 Kohlenstoffatomen, durch Fluor, Chlor, Brom, Trifluormethyl, Trifluormethoxy, Trifluormethylthio, Nitro, Cyano, Alkoxycarbonyl mit bis zu 4 Kohlenstoffatomen, Phenylsulfonyl, Tolylsulfonyl, Alkylsulfonyl mit bis zu 4 Kohlenstoffatomen, Hydroxy oder durch eine Gruppe der Formel

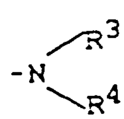 ,

in welcher

$R^3$ und $R^4$ gleich oder verschieden sind und Wasserstoff mit bis zu 4 Kohlenstoffatomen, Phenyl, Benzyl oder Acetyl bedeuten,

oder

- für Furyl, Thienyl oder Pyridyl steht, oder
- für geradkettiges, verzweigtes oder cyclisches Alkyl mit bis zu 6 Kohlenstoffatomen steht, das substituiert sein kann durch Fluor, Chlor, Brom, Phenyl, Thienyl, Pyridyl, Furyl oder Alkoxy mit bis zu 4 Kohlenstoffatomen

und

$R^2$ - für geradkettiges oder verzweigtes Alkyl mit bis zu 4 Kohlenstoffatomen steht,

in welchen das Ringkohlenstoffatom in 5-Stellung S-Konfiguration hat und in welchen der Substituent $R^1$ in 4-Stellung zum Substituenten $COOR^2$ in 5-Stellung cis konfiguriert ist.

**4.** Verfahren zur Herstellung enantiomerenreiner 4,5-disubstituierter γ-Butyrolactame der allgemeinen Formel (I) in welcher

$R^1$ - für Aryl mit 6 bis 12 Kohlenstoffatomen steht, das bis zu 5-fach gleich oder verschieden substituiert sein kann durch Alkyl, Alkoxy, Alkylthio mit jeweils bis zu 8 Kohlenstoffatomen, durch Aryl, Aryloxy, Arylthio mit jeweils 6 bis 12 Kohlenstoffatomen, durch Aralkyl mit 7 bis 14 Kohlenstoffatomen durch Halogen, Trifluormethyl, Trifluormethoxy, Difluormethoxy, Trifluormethylthio, Nitro, Cyano, Carboxy, Alkoxycarbonyl mit bis zu 8 Kohlenstoffatomen, Sulfo, Phenylsulfonyl, Tolylsulfonyl, Alkyl sulfonyl mit bis zu 8 Kohlenstoffatomen, Hydroxy oder durch eine Gruppe der Formel

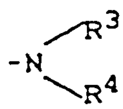,

worin

$R^3$ und $R^4$ gleich oder verschieden sind und Wasserstoff, Alkyl mit bis zu 8 Kohlenstoffatomen, Aryl mit 6 bis 12 Kohlenstoffatomen, Aralkyl mit 7 bis 14 Kohlenstoffatomen, Acyl mit 2 bis 7 Kohlenstoffatomen, Alkylsulfonyl mit bis zu 6 Kohlenstoffatomen, Phenylsulfonyl oder Tolylsulfonyl bedeuten,

oder

- für einen heterocyclischen Ring der Reihe Furyl, Thienyl, Pyridyl, Pyrimidyl, Pyrazinyl, Pyridazinyl, Chinolyl oder Isochinolyl steht, wobei diese Ringe bis zu 3-fach gleich oder verschieden substituiert sein können durch Alkyl, Alkoxy, Alkylthio mit jeweils bis zu 6 Kohlenstoffatomen, Halogen, Phenyl, Nitro, Cyano oder durch eine Gruppe der Formel

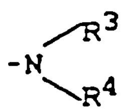,

worin
$R^3$ und $R^4$ die oben angegene Bedeutung haben,

oder

- für geradkettiges, verzweigtes oder cyclisches Alkyl oder Alkenyl mit bis zu 10 Kohlenstoffatomen steht, das substituiert sein kann durch Halogen, Aryl mit 6 bis 12 Kohlenstoffatomen, Furyl, Thienyl, Pyridyl, Pyrimidyl, Pyrazinyl, Pyridazinyl, Chinolyl, Isochinolyl, Hydroxy, Alkoxy, Alkylthio mit jeweils bis zu 6 Kohlenstoffatomen, Carboxy, Alkoxycarbonyl mit bis zu 6 Kohlenstoffatomen, Sulfo, Alkylsulfonyl mit bis zu 6 Kohlenstoffatomen, Phenylsulfonyl, Tolylsulfonyl oder durch eine Gruppe der Formel

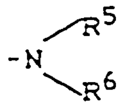 ,

worin

$R^5$ und $R^6$ gleich oder verschieden sind und Wasserstoff, Alkyl mit bis zu 8 Kohlenstoffatomen, Aryl mit 6 bis 12 Kohlenstoffatomen, Aralkyl mit 7 bis 14 Kohlenstoffatomen, Acyl mit bis zu 7 Kohlenstoffatomen, Alkylsulfonyl mit bis zu 6 Kohlenstoffatomen, Phenylsulfonyl oder Tolylsulfonyl bedeuten,

oder worin

$R^5$ und $R^6$ gemeinsam mit dem Stickstoffatom einen Ring aus der Reihe Pyrrolidino, Piperidino, Piperazino, Morpholino oder Thiomorpholino bilden und wobei dieser Ring substituiert sein kann durch Alkyl mit bis zu 4 Kohlenstoffatomen oder durch Phenyl

und

$R^2$ - für geradkettiges, verzweigtes oder cyclisches Alkyl mit bis zu 8 Kohlenstoffatomen steht,

in welchen das Ringkohlenstoffatom 5-Stellung S-Konfiguration hat und in welchen der Substituent $R^1$ in 4-Stellung zum Substituenten $COOR^2$ in 5-Stellung cis konfiguriert ist,
dadurch gekennzeichnet, daß man
Dihdropyrazine der allgemeinen Formel (II)

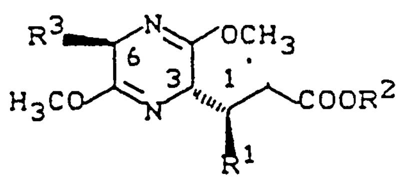 (II)

in welcher
$R^1$ und $R^2$ die angegebene Bedeutung haben,
und

$R^3$ - für geradkettiges oder verzweigtes Alkyl mit bis zu 4 Kohlenstoffatomen steht,

und in welcher das Kohlenstoffatom 6 des Dihydropyrazinrings in (II) R-konfiguriert ist, das Kohlenstoffatom 3 des Pyrazinrings in (II) S-konfiguriert ist und das Kohlenstoffatom in 1' S-konfiguriert ist, wenn $R^1$ die höhere Priorität hat als die Gruppe $CH_2COOR^2$ oder R-konfiguriert ist, wenn $R^1$ eine niedrigere Priorität hat als die Gruppe $CH_2COOR^2$
zunächst in inerten Lösemitteln mit Säuren hydrolysiert,
dann aus den entstehenden sauren Aminosäuresalzen mit Basen in inerten Lösemitteln die freien Aminosäuren herstellt
und diese anschließend cyclisiert.

**5.** Dihydropyrazine der allgemeinen Formel (II)

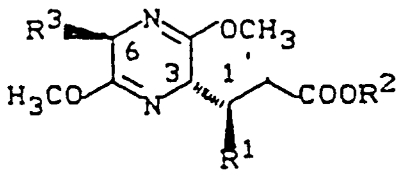

(II)

in welcher

$R^1$ - für Aryl mit 6 bis 14 Kohlenstoffatomen steht, das bis zu 5-fach gleich oder verschieden substituiert sein kann durch Alkyl, Alkoxy, Alkylthio mit jeweils bis zu 8 Kohlenstoffatomen, durch Aryl, Aryloxy, Arylthio mit jeweils 6 bis 12 Kohlenstoffatomen, durch Aralkyl mit 7 bis 14 Kohlenstoffatomen,durch Halogen, Trifluormethyl, Trifluormethoxy, Difluormethoxy, Trifluormethylthio, Nitro, Cyano, Carboxy, Alkoxycarbonyl mit bis zu 8 Kohlenstoffatomen, Sulfo, Phenylsulfonyl, Tolylsulfonyl, Alkylsulfonyl mit bis zu 8 Kohlenstoffatomen, Hydroxy oder durch eine Gruppe der Formel

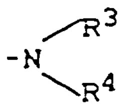

worin

$R^3$ und $R^4$ gleich oder verschieden sind und Wasserstoff, Alkyl mit bis zu 8 Kohlenstoffatomen, Aryl mit 6 bis 12 Kohlenstoffatomen, Aralkyl mit 7 bis 14 Kohlenstoffatomen, Acyl mit bis zu 7 Kohlenstoffatomen, Alkylsulfonyl mit bis zu 6 Kohlenstoffatomen, Phenylsulfonyl oder Tolylsulfonyl bedeuten, oder

- für einen heterocyclischen Ring der Reihe Furyl, Thienyl, Pyridyl, Pyrimidyl, Pyrazinyl, Pyridazinyl, Chinolyl oder Isochinolyl steht, wobei diese Ringe bis zu 3-fach gleich oder verschieden substituiert sein können durch Alkyl, Alkoxy, Alkylthio mit jeweils bis zu 6 Kohlenstoffatomen, Halogen, Phenyl, Nitro, Cyano oder durch eine Gruppe der Formel

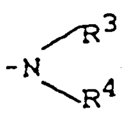

,

worin
$R^3$ und $R^4$ die oben angegebene Bedeutung haben,

oder

- für geradkettiges, verzweigtes oder cyclisches Alkyl oder Alkenyl mit bis zu 10 Kohlenstoffatomen steht, das substituiert sein kann durch Halogen, Aryl mit 6 bis 12 Kohlenstoffatomen, Furyl, Thienyl, Pyridyl, Pyrimidyl, Pyrazinyl, Pyridazinyl, Chinolyl, Isochinolyl, Hydroxy, Alkoxy, Alkylthio mit jeweils bis zu 6 Kohlenstoffatomen, Carboxy, Alkoxycarbonyl mit bis zu 6 Kohlenstoffatomen, Sulfo, Alkylsulfonyl mit bis zu 6 Kohlenstoffatomen, Phenylsulfonyl, Tolylsulfonyl oder
durch eine Gruppe der Formel

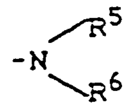,

worin

| | |
|---|---|
| $R^5$ und $R^6$ | gleich oder verschieden sind und Wasserstoff, Alkyl mit bis zu 8 Kohlenstoffatomen, Aryl mit 6 bis 12 Kohlenstoffatomen, Aralkyl mit 7 bis 14 Kohlenstoffatomen, Acyl mit bis zu 7 Kohlenstoffatomen, Alkylsulfonyl mit bis zu 6 Kohlenstoffatomen, Phenylsulfonyl oder Tolylsulfonyl bedeuten, |

oder worin

| | |
|---|---|
| $R^5$ und $R^6$ | gemeinsam mit dem Stickstoffatom einen Ring aus der Reihe Pyrrolidino, Piperidino, Piperazino, Morpholino oder Thiomorpholino bilden und wobei dieser Ring substituiert sein kann durch Alkyl mit bis zu 4 Kohlenstoffatomen oder durch Phenyl, |

$R^2$ - für geradkettiges, verzweigtes oder cyclisches Alkyl mit bis zu 8 Kohlenstoffatomen steht,

und

$R^3$ - für geradkettiges oder verzweigtes Alkyl mit bis zu 4 Kohlenstoffatomen steht,

und worin das Kohlenstofatom 3 des Dihydropyrazinrings S-konfiguriert und das Kohlenstoffatom 6 R-konfiguriert ist, das Kohlenstoffatom 1' in der Seitenkette S-konfiguriert ist, wenn $R^1$ die höhere Priorität hat als die Gruppe $CH_2COOR^2$ oder R-konfiguriert ist, wenn $R^1$ eine niedrigere Priorität hat als die Gruppe $CH_2COOR^2$.

6. Verfahren zur Herstellung von Dihydropyrazinen der allgemeinen Formel II in Anspruch 5, dadurch gekennzeichnet, daß man Verbindungen der Formel (III)

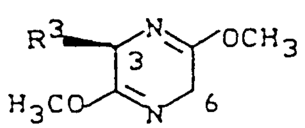

(III),

in welcher
$R^3$ die oben angegebene Bedeutung hat und das Kohlenstoffatom des Dihydropyrazinrings R-konfiguriert ist, zunächst mit einer stark basischen metallorganischen Verbindung zu den in 6-Stellung durch das Metall der metallorganischen Verbindungen monosubstituierten Derivaten von (III) umsetzt,
dann diese Metallderivate mit einem cis-substituierten Acrylester der allgemeinen Formel (IV)

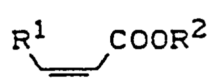

(IV),

in welcher
$R^1$ und $R^2$ die oben angegebene Bedeutung haben, in inerten Lösemitteln umsetzt und anschließend mit einer Säure neutralisiert.

**Patentansprüche für folgenden Vertragsstaat : ES**

1. Verfahren zur Herstellung von enantiomerenreinen 4,5-disubstituierten $\gamma$-Butyrolactamen der allgemeinen Formel (I)

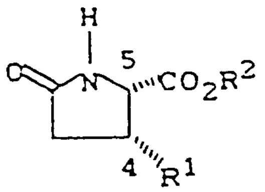

(I),

in welcher

$R^1$ - für Aryl mit 6 bis 14 Kohlenstoffatomen steht, das bis zu 5-fach gleich oder verschieden substituiert sein kann durch Alkyl, Alkoxy, Alkylthio mit jeweils bis zu 8 Kohlenstoffatomen, durch Aryl, Aryloxy, Arylthio mit jeweils 6 bis 12 Kohlenstoffatomen, durch Aralkyl mit 7 bis 14 Kohlenstoffatomen durch Halogen, Trifluormethyl, Trifluormethoxy, Difluormethoxy, Trifluormethylthio, Nitro, Cyano, Carboxy, Alkoxycarbonyl mit bis zu 8 Kohlenstoffatomen, Sulfo, Phenylsulfonyl, Tolylsulfonyl, Alkylsulfonyl mit bis zu 8 Kohlenstoffatomen, Hydroxy oder durch eine Gruppe der Formel

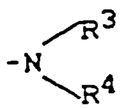

,

worin

$R^3$ und $R^4$ gleich oder verschieden sind und Wasserstoff, Alkyl mit bis zu 8 Kohlenstoffatomen, Aryl mit 6 bis 12 Kohlenstoffatomen, Aralkyl mit 7 bis 14 Kohlenstoffatomen, Acyl mit bis zu 7 Kohlenstoffatomen, Alkylsulfonyl mit bis zu 6 Kohlenstoffatomen, Phenylsulfonyl oder Tolylsulfonyl bedeuten,

oder

- für einen heterocyclischen Ring der Reihe Furyl, Thienyl, Pyridyl, Pyrimidyl, Pyrazinyl, Pyridazinyl, Chinolyl oder Isochinolyl steht, wobei diese Ringe bis zu 3-fach gleich oder verschieden substituiert sein können durch Alkyl, Alkoxy, Alkylthio mit jeweils bis zu 6 Kohlenstoffatomen, Halogen, Phenyl, Nitro, Cyano oder durch eine Gruppe der Formel

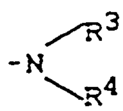

,

worin
$R^3$ und $R^4$ die oben angegebene Bedeutung haben,

oder

- für geradkettiges, verzweigtes oder cyclisches Alkyl oder Alkenyl mit bis zu 10 Kohlenstoffatomen steht, das substituiert sein kann durch Halogen, Aryl mit 6 bis 14 Kohlenstoffatomen, Furyl, Thienyl, Pyridyl, Pyrimidyl, Pyrazinyl, Pyridazinyl, Chinolyl, Isochinolyl, Hydroxy, Alkoxy, Alkylthio mit jeweils bis zu 6 Kohlenstoffatomen, Carboxy, Alkoxycarbonyl mit bis zu 6 Kohlenstoffatomen, Sulfo, Alkylsulfonyl mit bis zu 6 Kohlenstoffatomen, Phenylsulfonyl, Tolylsulfonyl oder
durch eine Gruppe der Formel

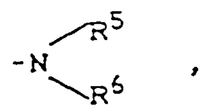

worin

$R^5$ und $R^6$ gleich oder verschieden sind und Wasserstoff, Alkyl mit bis zu 8 Kohlenstoffatomen, Aryl mit 6 bis 12 Kohlenstoffatomen, Aralkyl mit 7 bis 14 Kohlenstoffatomen, Acyl mit bis zu 7 Kohlenstoffatomen, Alkylsulfonyl mit bis zu 6 Kohlenstoffatomen, Phenylsulfonyl oder Tolylsulfonyl bedeuten,

oder worin

$R^5$ und $R^6$ gemeinsam mit dem Stickstoffatom einen Ring aus der Reihe Pyrrolidino, Piperidino, Piperazino, Morpholino oder Thiomorpholino bilden und wobei dieser Ring substituiert sein kann durch Alkyl mit bis zu 4 Kohlenstoffatomen oder durch Phenyl

und

$R^2$ - für geradkettiges, verzweigtes oder cyclisches Alkyl mit bis zu 8 Kohlenstoffatomen steht,

in welchen das Ringkohlenstoffatom 5-Stellung S-Konfiguration hat und in welchen der Substituent $R^1$ in 4-Stellung zum Substituenten $COOR^2$ in 5-Stellung cis konfiguriert ist,
dadurch gekennzeichnet, daß man
Dihdropyrazine der allgemeinen Formel (II)

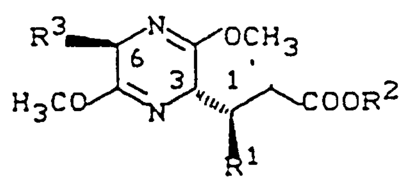

(II)

in welcher
$R^1$ und $R^2$ die angegebene Bedeutung haben,
und

$R^3$ - für geradkettiges oder verzweigtes Alkyl mit bis zu 4 Kohlenstoffatomen steht,

und in welcher das Kohlenstoffatom 6 des Dihydropyrazinrings in (II) R-konfiguriert ist, das Kohlenstoffatom 3 des Pyrazinrings in (II) S-konfiguriert ist und das Kohlenstoffatom in 1' S-konfiguriert ist, wenn $R^1$ die höhere Priorität hat als die Gruppe $CH_2COOR^2$ oder R-konfiguriert ist, wenn $R^1$ eine niedrigere Priorität hat als die Gruppe $CH_2COOR^2$
zunächst in inerten Lösemitteln mit Säuren hydrolysiert,
dann aus den entstehenden sauren Aminosäuresalzen mit Basen in inerten Lösemitteln die freien Aminosäuren herstellt
und diese anschließend cyclisiert.

**2.** Verfahren zur Herstellung von Dihydropyrazinen der allgemeinen Formel (II)

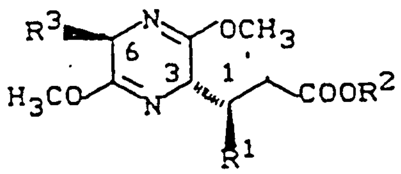

(II)

in welcher

$R^1$ - für Aryl mit 6 bis 14 Kohlenstoffatomen steht, das bis zu 5-fach gleich oder verschieden substituiert sein kann durch Alkyl, Alkoxy, Alkylthio mit jeweils bis zu 8 Kohlenstoffatomen, durch Aryl, Aryloxy, Arylthio mit jeweils 6 bis 12 Kohlenstoffatomen, durch Aralkyl mit 7 bis 14 Kohlenstoffatomen, durch Halogen, Trifluormethyl, Trifluormethoxy, Difluormethoxy, Trifluormethylthio, Nitro, Cyano, Carboxy, Alkoxycarbonyl mit bis zu 8 Kohlenstoffatomen, Sulfo, Phenylsulfonyl, Tolylsulfonyl, Alkylsulfonyl mit bis zu 8 Kohlenstoffatomen, Hydroxy oder durch eine Gruppe der Formel

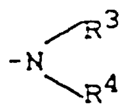

worin

$R^3$ und $R^4$ gleich oder verschieden sind und Wasserstoff, Alkyl mit bis zu 8 Kohlenstoffatomen, Aryl mit 6 bis 12 Kohlenstoffatomen, Aralkyl mit 7 bis 14 Kohlenstoffatomen, Acyl mit bis zu 7 Kohlenstoffatomen, Alkylsulfonyl mit bis zu 6 Kohlenstoffatomen, Phenylsulfonyl oder Tolylsulfonyl bedeuten,

oder

- für einen heterocyclischen Ring der Reihe Furyl, Thienyl, Pyridyl, Pyrimidyl, Pyrazinyl, Pyridazinyl, Chinolyl oder Isochinolyl steht, wobei diese Ringe bis zu 3-fach gleich oder verschieden substituiert sein können durch Alkyl, Alkoxy, Alkylthio mit jeweils bis zu 6 Kohlenstoffatomen, Halogen, Phenyl, Nitro, Cyano oder durch eine Gruppe der Formel

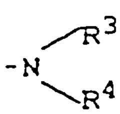 ,

  worin
  $R^3$ und $R^4$ die oben angegebene Bedeutung haben,

oder

- für geradkettiges, verzweigtes oder cyclisches Alkyl oder Alkenyl mit bis zu 10 Kohlenstoffatomen steht, das substituiert sein kann durch Halogen, Aryl mit 6 bis 12 Kohlenstoffatomen, Furyl, Thienyl, Pyridyl, Pyrimidyl, Pyrazinyl, Pyridazinyl, Chinolyl, Isochinolyl, Hydroxy, Alkoxy, Alkylthio mit jeweils bis zu 6 Kohlenstoffatomen, Carboxy, Alkoxycarbonyl mit bis zu 6 Kohlenstoffatomen, Sulfo, Alkylsulfonyl mit bis zu 6 Kohlenstoffatomen, Phenylsulfonyl, Tolylsulfonyl oder
  durch eine Gruppe der Formel

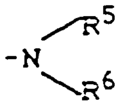 ,

worin

$R^5$ und $R^6$ gleich oder verschieden sind und Wasserstoff, Alkyl mit bis zu 8 Kohlenstoffatomen, Aryl mit 6 bis 12 Kohlenstoffatomen, Aralkyl mit 7 bis 14 Kohlenstoffatomen, Acyl mit bis zu 7 Kohlenstoffatomen, Alkylsulfonyl mit bis zu 6 Kohlenstoffatomen, Phenylsulfonyl oder Tolylsulfonyl bedeuten,

oder worin

$R^5$ und $R^6$ gemeinsam mit dem Stickstoffatom einen Ring aus der Reihe Pyrrolidino, Piperidino, Piperazino, Morpholino oder Thiomorpholino bilden und wobei dieser Ring substituiert sein kann durch Alkyl mit bis zu 4 Kohlenstoffatomen oder durch Phenyl,

$R^2$ - für geradkettiges, verzweigtes oder cyclisches Alkyl mit bis zu 8 Kohlenstoffatomen steht,

und

$R^3$ - für geradkettiges oder verzweigtes Alkyl mit bis zu 4 Kohlenstoffatomen steht,

und worin das Kohlenstofatom 3 des Dihydropyrazinrings S-konfiguriert und das Kohlenstoffatom 6 R-konfiguriert ist, das Kohlenstoffatom 1' in der Seitenkette S-konfiguriert ist, wenn $R^1$ die höhere Priorität hat als die Gruppe $CH_2COOR^2$ oder R-konfiguriert ist, wenn $R^1$ eine niedrigere Priorität hat als die Gruppe $CH_2COOR^2$, dadurch gekennzeichnet, daß man Verbindungen der Formel (III)

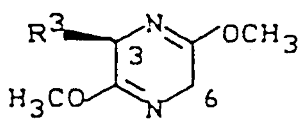 (III),

in welcher
$R^3$ die oben angegebene Bedeutung hat und das Kohlenstoffatom des Dihydropyrazinrings R-konfiguriert ist, zunächst mit einer stark basischen metallorganischen Verbindung zu den in 6-Stellung durch das Metall der metallorganischen Verbindungen monosubstituierten Derivaten von (III) umsetzt,
dann diese Metallderivate mit einem cis-substituierten Acrylester der allgemeinen Formel (IV)

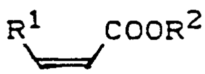 (IV),

in welcher
$R^1$ und $R^2$ die oben angegebene Bedeutung haben, in inerten Lösemitteln umsetzt und anschließend mit einer Säure neutralisiert.

## Claims

**Claims for the following Contracting States : AT, BE, CH, DE, FR, GB, IT, LI, NL, SE**

1. Pure enantiomers of 4,5-disubstituted $\gamma$-butyrolactams of the general formula (I)

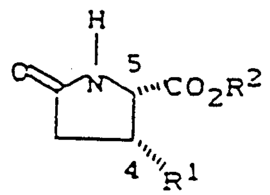

(I),

in which

$R^1$ represents aryl which has 6 to 14 carbon atoms and can be up to pentasubstituted by identical or different substituents from amongst alkyl, alkoxy and alkylthio each having up to 8 carbon atoms, aryl, aryloxy and arylthio each having 6 to 12 carbon atoms, aralkyl having 7 to 14 carbon atoms, halogen, trifluoromethyl, trifluoromethoxy, difluoromethoxy, trifluoromethylthio, nitro, cyano, carboxyl, alkoxycarbonyl having up to 8 carbon atoms, sulpho, phenylsulphonyl, tolylsulphonyl, alkylsulphonyl having up to 8 carbon atoms, hydroxyl or a group of the formula

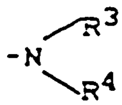

,

wherein

$R^3$ and $R^4$ are identical or different and denote hydrogen, alkyl having up to 8 carbon atoms, aryl having 6 to 12 carbon atoms, aralkyl having 7 to 14 carbon atoms, acyl having up to 7 carbon atoms, alkylsulphonyl having up to 6 carbon atoms, phenylsulphonyl or tolylsulphonyl,

or

represents a heterocyclic ring from the group comprising furyl, thienyl, pyridyl, pyrimidyl, pyrazinyl, pyridazinyl, quinolyl and isoquinolyl, it being possible for these rings to be up to trisubstituted by identical or different substituents from amongst alkyl, alkoxy and alkylthio each having up to 6 carbon atoms, halogen, phenyl, nitro, cyano or a group of the formula

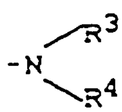

,

wherein
$R^3$ and $R^4$ have the meaning given above,

or

represents straight-chain, branched or cyclic alkyl or alkenyl which has up to 10 carbon atoms and can be substituted by halogen, aryl having 6 to 14 carbon atoms, furyl, thienyl, pyridyl, pyrimidyl, pyrazinyl, pyridazinyl, quinolyl, isoquinolyl, hydroxyl, alkoxy or alkylthio each having up to 6 carbon atoms, carboxyl, alkoxycarbonyl having up to 6 carbon atoms, sulpho, alkylsulphonyl having up to 6 carbon atoms, phenylsulphonyl, tolylsulphonyl or by a group of the formula

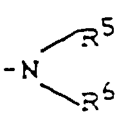

,

wherein

$R^5$ and $R^6$ are identical or different and denote hydrogen, alkyl having up to 8 carbon atoms, aryl having 6 to 12 carbon atoms, aralkyl having 7 to 14 carbon atoms, acyl having up to 7 carbon atoms, alkylsulphonyl having up to 6 carbon atoms, phenylsulphonyl or tolylsulphonyl,

or wherein

$R^5$ and $R^6$ together with the nitrogen atom form a ring from the group comprising pyrrolidino, piperidino, piperazino, morpholino and thiomorpholino, it being possible for this ring to be substituted by alkyl having up to 4 carbon atoms or by phenyl,

and

$R^2$ represents straight-chain, branched or cyclic alkyl having up to 8 carbon atoms,

in which the ring carbon atom in the 5-position has the S-configuration and in which the substituent $R^1$ in the 4-position has the cis-configuration relative to the substituent $COOR^2$ in the 5-position.

**2.** Compounds of the general formula (I) in Claim 1, in which

$R^1$ represents phenyl or naphthyl which can be up to trisubstituted by identical or different substituents from amongst alkyl and alkoxy each having up to 6 carbon atoms, methylthio, phenyl, phenoxy, benzyl, fluoro, chloro, bromo, iodo, trifluoromethyl, trifluoromethoxy, difluoromethoxy, trifluoromethylthio, nitro, cyano, alkoxycarbonyl having up to 6 carbon atoms, phenylsulphonyl, tolylsulphonyl, alkylsulphonyl having up to 6 carbon atoms, hydroxyl or a group of the formula

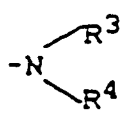 ,

in which

$R^3$ and $R^4$ are identical or different and denote hydrogen, alkyl having up to 6 carbon atoms, phenyl, benzyl, acetyl, ethylcarbonyl, benzoyl, alkylsulphonyl having up to 4 carbon atoms, tolylsulphonyl or phenylsulphonyl,

or

represents a heterocyclic ring from the group comprising furyl, thienyl, pyridyl, pyrimidyl, quinolyl and isoquinolyl, it being possible for these rings to be substituted by alkyl or alkoxy having up to 4 carbon atoms, by fluoro, chloro, bromo, nitro, cyano or by a group of the formula

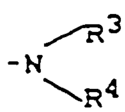 ,

wherein

$R^3$ and $R^4$ have the meaning given above,

or

represents straight-chain, branched or cyclic alkyl which has up to 8 carbon atoms and can be substituted by fluoro, chloro, bromo, phenyl, furyl, thienyl, pyridyl, pyrimidyl, quinolyl, isoquinolyl, hydroxyl, alkoxy or alkylthio having up to 4 carbon atoms, alkoxycarbonyl having up to 4 carbon atoms, alkylsulphonyl having up to 4 carbon atoms, phenylsulphonyl, tolylsulphonyl or by a group of the formula

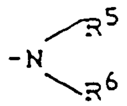,

wherein

$R^5$ and $R^6$ are identical or different and denote hydrogen, alkyl having up to 6 carbon atoms, phenyl, benzyl, acetyl, ethylcarbonyl, benzoyl, alkylsulphonyl having up to 4 carbon atoms, tolylsulphonyl or phenylsulphonyl,

or in which

$R^5$ and $R^6$ together with the nitrogen atom form a ring from the group comprising pyrrolidino, piperidino, N-methyl- or N-phenylpiperazino and morpholino,

and

$R^2$ represents straight-chain or branched alkyl having up to 6 carbon atoms,

and in which the ring carbon atom in the 5-position has the S-configuration and in which the substituent $R^1$ in the 4-position has the cis-configuration relative to the substituent $COOR^2$ in the 5-position.

**3.** Compounds of the general formula (I) in Claim 1, in which

$R^1$ represents phenyl which can be substituted by alkyl or alkoxy having up to 4 carbon atoms, by fluoro, chloro, bromo, trifluoromethyl, trifluoromethoxy, trifluoromethylthio, nitro, cyano, alkoxycarbonyl having up to 4 carbon atoms, phenylsulphonyl, tolylsulphonyl, alkylsulphonyl having up to 4 carbon atoms, hydroxyl or by a group of the formula

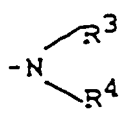,

in which

$R^3$ and $R^4$ are identical or different and denote hydrogen, phenyl, benzyl or acetyl,

or

represents furyl, thienyl or pyridyl, or represents straight-chain, branched or cyclic alkyl which has up to 6 carbon atoms and can be substituted by fluoro, chloro, bromo, phenyl, thienyl, pyridyl, furyl or alkoxy having up to 4 carbon atoms

and

$R^2$ represents straight-chain or branched alkyl having up to 4 carbon atoms,

in which the ring carbon atom in the 5-position has the S-configuration and in which the substituent $R^1$ in the 4-position has the cis-configuration relative to the substituent $COOR^2$ in the 5-position.

**4.** Process for the preparation of pure enantiomers of 4,5-disubstituted $\gamma$-butyrolactams of the general formula (I) in which

$R^1$ represents aryl which has 6 to 12 carbon atoms and can be up to pentasubstituted by identical or different substituents from amongst alkyl, alkoxy and alkylthio each having up to 8 carbon atoms, aryl, aryloxy and arylthio each having 6 to 12 carbon atoms, aralkyl having 7 to 14 carbon atoms, halogen, trifluoromethyl, trifluoromethoxy, difluoromethoxy, trifluoromethylthio, nitro, cyano, carboxyl, alkoxycarbonyl having up to 8 carbon atoms, sulpho, phenylsulphonyl, tolylsulphonyl, alkylsulphonyl having up to 8 carbon atoms. hydroxyl or a group of the formula

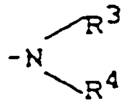 ,

wherein

$R^3$ and $R^4$ are identical or different and denote hydrogen, alkyl having up to 8 carbon atoms, aryl having 6 to 12 carbon atoms, aralkyl having 7 to 14 carbon atoms, acyl having 2 to 7 carbon atoms, alkylsulphonyl having up to 6 carbon atoms, phenylsulphonyl or tolylsulphonyl,

or

represents a heterocyclic ring from the group comprising furyl, thienyl, pyridyl, pyrimidyl, pyrazinyl, pyridazinyl, quinolyl and isoquinolyl, it being possible for these rings to be up to trisubstituted by identical or different substituents from amongst alkyl, alkoxy and alkylthio each having up to 6 carbon atoms, halogen, phenyl, nitro, cyano or a group of the formula

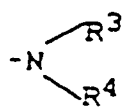 ,

wherein
$R^3$ and $R^4$ have the meaning given above,
or
represents straight-chain, branched or cyclic alkyl or alkenyl which has up to 10 carbon atoms and can be substituted by halogen, aryl having 6 to 12 carbon atoms, furyl, thienyl, pyridyl, pyrimidyl, pyrazinyl, pyridazinyl, quinolyl, isoquinolyl, hydroxyl, alkoxy or alkylthio each having up to 6 carbon atoms, carboxyl, alkoxycarbonyl having up to 6 carbon atoms, sulpho, alkylsulphonyl having up to 6 carbon atoms, phenylsulphonyl, tolylsulphonyl or by a group of the formula

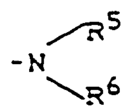 ,

wherein

$R^5$ and $R^6$ are identical or different and denote hydrogen, alkyl having up to 8 carbon atoms, aryl having 6 to 12 carbon atoms, aralkyl having 7 to 14 carbon atoms, acyl having up to 7 carbon atoms, alkylsulphonyl having up to 6 carbon atoms, phenylsulphonyl or tolylsulphonyl,

or wherein

$R^5$ and $R^6$ together with the nitrogen atom form a ring from the group comprising pyrrolidino, piperidino, piperazino, morpholino and thiomorpholino, it being possible for this ring to be substituted by alkyl having up to 4 carbon atoms or by phenyl,

and

$R^2$ represents straight-chain, branched or cyclic alkyl having up to 8 carbon atoms,

in which the ring carbon atom in the 5-position has the S-configuration and in which the substituent $R^1$ in the 4-position has the cis-configuration relative to the substituent $COOR^2$ in the 5-position,

characterized in that
dihydropyrazines of the general formula (II)

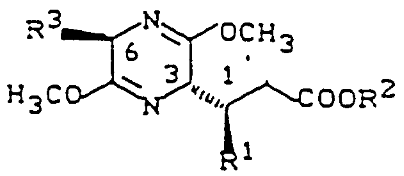
(II)

in which
$R^1$ and $R^2$ have the meaning given,
and

$R^3$ represents straight-chain or branched alkyl having up to 4 carbon atoms,

and in which the carbon atom 6 of the dihydropyrazine ring in (II) has the R-configuration, the carbon atom 3 of the pyrazine ring in (II) has the S-configuration and the carbon atom 1' has the S-configuration, if $R^1$ has a higher priority than the group $CH_2COOR^2$, or has the R-configuration, if $R^1$ has a lower priority than the group $CH_2COOR^2$,

are first hydrolysed with acids in inert solvents, then the free amino acids are prepared from the resulting acidic amino acid salts with bases in inert solvents and the free amino acids are subsequently cyclized.

**5.** Dihydropyrazines of the general formula (II)

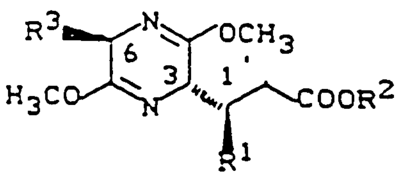
(II)

in which

$R^1$ represents aryl which has 6 to 14 carbon atoms and can be up to pentasubstituted by identical or different substituents from amongst alkyl, alkoxy and alkylthio each having up to 8 carbon atoms, aryl, aryloxy and arylthio each having 6 to 12 carbon atoms, aralkyl having 7 to 14 carbon atoms, halogen, trifluoromethyl, trifluoromethoxy, difluoromethoxy, trifluoromethylthio, nitro, cyano, carboxyl, alkoxycarbonyl having up to 8 carbon atoms, sulpho, phenylsulphonyl, tolylsulphonyl, alkylsulphonyl having up to 8 carbon atoms, hydroxyl or a group of the formula

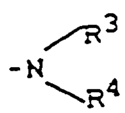

wherein

$R^3$ and $R^4$ are identical or different and denote hydrogen, alkyl having up to 8 carbon atoms, aryl having 6 to 12 carbon atoms, aralkyl having 7 to 14 carbon atoms, acyl having up to 7 carbon atoms, alkylsulphonyl having up to 6 carbon atoms, phenylsulphonyl or tolylsulphonyl,

or

represents a heterocyclic ring from the group comprising furyl, thienyl, pyridyl, pyrimidyl, pyrazinyl, pyridazinyl, quinolyl and isoquinolyl, it being possible for these rings to be up to trisubstituted by identical or different substituents from amongst alkyl, alkoxy and alkylthio each having up to 6 carbon atoms, halogen, phenyl, nitro, cyano or a group of the formula

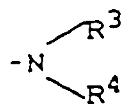 ,

wherein
$R^3$ and $R^4$ have the meaning given above,

or

represents straight-chain, branched or cyclic alkyl or alkenyl which has up to 10 carbon atoms and can be substituted by halogen, aryl having 6 to 12 carbon atoms, furyl, thienyl, pyridyl, pyrimidyl, pyrazinyl, pyridazinyl, quinolyl, isoquinolyl, hydroxyl, alkoxy or alkylthio each having up to 6 carbon atoms, carboxyl, alkoxycarbonyl having up to 6 carbon atoms, sulpho, alkylsulphonyl having up to 6 carbon atoms, phenylsulphonyl, tolylsulphonyl or by a group of the formula

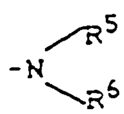 ,

wherein

$R^5$ and $R^6$ are identical or different and denote hydrogen, alkyl having up to 8 carbon atoms, aryl having 6 to 12 carbon atoms, aralkyl having 7 to 14 carbon atoms, acyl having up to 7 carbon atoms, alkylsulphonyl having up to 6 carbon atoms, phenylsulphonyl or tolylsulphonyl,

or wherein

$R^5$ and $R^6$ together with the nitrogen atom form a ring from the group comprising pyrrolidino, piperidino, piperazino, morpholino and thiomorpholino, it being possible for this ring to be substituted by alkyl having up to 4 carbon atoms or by phenyl,

$R^2$ represents straight-chain, branched or cyclic alkyl having up to 8 carbon atoms,

and

$R^3$ represents straight-chain or branched alkyl having up to 4 carbon atoms,

and wherein the carbon atom 3 of the dihydropyrazine ring has the S-configuration and the carbon atom 6 has the R-configuration, the carbon atom 1' in the side chain has the S-configuration, if $R^1$ has a higher priority than the group $CH_2COOR^2$, or has the R-configuration, if $R^1$ has a lower priority than the group $CH_2COOR^2$.

**6.** Process for the preparation of dihydropyrazines of the general formula II in Claim 5, characterized in that compounds of the formula (III)

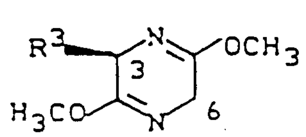 (III),

in which
$R^3$ has the meaning given above and the carbon atom of the dihydropyrazine ring has the R-configuration,
are first reacted with a strongly basic metal-organic compound to give the derivatives of (III) which are monosubstituted in the 6-position by the metal of the metal-organic compounds,
these metal derivatives are then reacted in inert solvents with a cis-substituted acrylic ester of the general formula

(IV)

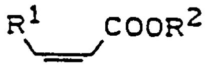

(IV),

in which
$R^1$ and $R^2$ have the meaning given above, and subsequently neutralized with an acid.

**Claims for the following Contracting State : ES**

1. Process for the preparation of pure enantiomers of 4,5-disubstituted γ-butyrolactams of the general formula (I)

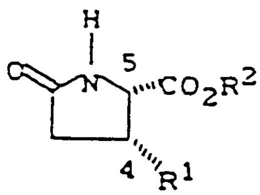

(I),

in which

$R^1$ represents aryl which has 6 to 14 carbon atoms and can be up to pentasubstituted by identical or different substituents from amongst alkyl, alkoxy and alkylthio each having up to 8 carbon atoms, aryl, aryloxy and arylthio each having 6 to 12 carbon atoms, aralkyl having 7 to 14 carbon atoms, halogen, trifluoromethyl, trifluoromethoxy, difluoromethoxy, trifluoromethylthio, nitro, cyano, carboxyl, alkoxycarbonyl having up to 8 carbon atoms, sulpho, phenylsulphonyl, tolylsulphonyl, alkylsulphonyl having up to 8 carbon atoms, hydroxyl or a group of the formula

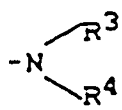

,

wherein

$R^3$ and $R^4$ are identical or different and denote hydrogen, all having up to 8 carbon atoms, aryl having 6 to 12 carbon atoms, aralkyl having 7 to 14 carbon atoms, acyl having up to 7 carbon atoms, alkylsulphonyl having up to 6 carbon atoms, phenylsulphonyl or tolylsulphonyl,

or

represents a heterocyclic ring from the group comprising furyl, thienyl, pyridyl, pyrimidyl, pyrazinyl, pyridazinyl, quinolyl and isoquinolyl, it being possible for these rings to be up to trisubstituted by identical or different substituents from amongst alkyl, alkoxy and alkylthio each having up to 6 carbon atoms, halogen, phenyl, nitro, cyano or a group of the formula

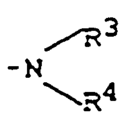

,

wherein
$R^3$ and $R^4$ have the meaning given above, or

represents straight-chain, branched or cyclic alkyl or alkenyl which has up to 10 carbon atoms and can be substituted by halogen, aryl having 6 to 14 carbon atoms, furyl, thienyl, pyridyl, pyrimidyl, pyrazinyl, pyridazinyl, quinolyl, isoquinolyl, hydroxyl, alkoxy or alkylthio each having up to 6 carbon atoms, carboxyl, alkoxycarbonyl having up to 6 carbon atoms, sulpho, alkylsulphonyl having up to 6 carbon atoms, phenylsulphonyl, tolylsulphonyl or by a group of the formula

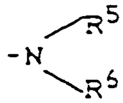 ,

wherein

$R^5$ and $R^6$ are identical or different and denote hydrogen, all having up to 8 carbon atoms, aryl having 6 to 12 carbon atoms, aralkyl having 7 to 14 carbon atoms, acyl having up to 7 carbon atoms, alkylsulphonyl having up to 6 carbon atoms, phenylsulphonyl or tolylsulphonyl,

or wherein

$R^5$ and $R^6$ together with the nitrogen atom form a ring from the group comprising pyrrolidino, piperidino, piperazino, morpholino and thiomorpholino, it being possible for this ring to be substituted by alkyl having up to 4 carbon atoms or by phenyl,

and

$R^2$ represents straight-chain, branched or cyclic alkyl having up to 8 carbon atoms,

in which the ring carbon atom in the 5-position has the S-configuration and in which the substituent $R^1$ in the 4-position has the cis-configuration relative to the substituent $COOR^2$ in the 5-position,
characterized in that
dihydropyrazines of the general formula (II)

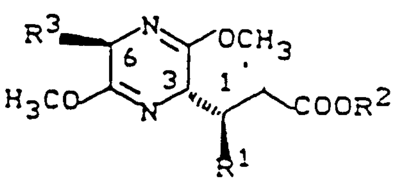

(II)

in which
$R^1$ and $R^2$ have the meaning given,
and

$R^3$ represents straight-chain or branched all having up to 4 carbon atoms,

and in which the carbon atom 6 of the dihydropyrazine ring in (II) has the R-configuration, the carbon atom 3 of the pyrazine ring in (II) has the S-configuration, and the carbon atom 1' has the S-configuration, if $R^1$ has a higher priority than the group $CH_2COOR^2$, or has the R-configuration, if $R^1$ has a lower priority than the group $CH_2COOR^2$, are first hydrolysed with acids in inert solvents, then the free amino acids are prepared from the resulting acidic amino acid salts with bases in inert solvents and the free amino acids are subsequently cyclized.

**2.** Process for the preparation of dihydropyrazines of the general formula (II)

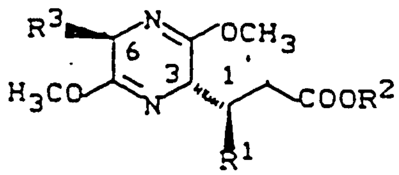

(II)

in which

$R^1$ represents aryl which has 6 to 14 carbon atoms and can be up to pentasubstituted by identical or different substituents from amongst alkyl, alkoxy and alkylthio each having up to 8 carbon atoms, aryl, aryloxy and arylthio each having 6 to 12 carbon atoms, aralkyl having 7 to 14 carbon atoms, halogen, trifluoromethyl, trifluoromethoxy, difluoromethoxy, trifluoromethylthio, nitro, cyano, carboxyl, alkoxycarbonyl having up to 8 carbon atoms, sulpho, phenylsulphonyl, tolylsulphonyl, alkylsulphonyl having up to 8 carbon atoms, hydroxyl or a group of the formula

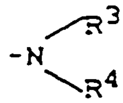

in which

$R^3$ and $R^4$ are identical or different and denote hydrogen, alkyl having up to 8 carbon atoms, aryl having 6 to 12 carbon atoms, aralkyl having 7 to 14 carbon atoms, acyl having up to 7 carbon atoms, alkylsulphonyl having up to 6 carbon atoms, phenylsulphonyl or tolylsulphonyl,

or

represents a heterocyclic ring from the group comprising furyl, thienyl, pyridyl, pyrimidyl, pyrazinyl, pyridazinyl, quinolyl and isoquinolyl, it being possible for these rings to be up to trisubstituted by identical or different substituents from amongst alkyl, alkoxy and alkylthio each having up to 6 carbon atoms, halogen, phenyl, nitro, cyano or a group of the formula

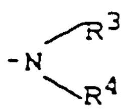

,

in which

$R^3$ and $R^4$ have the meaning given above, or

represents straight-chain, branched or cyclic alkyl or alkenyl which has up to 10 carbon atoms and can be substituted by halogen, aryl having 6 to 12 carbon atoms, furyl, thienyl, pyridyl, pyrimidyl, pyrazinyl, pyridazinyl, quinolyl, isoquinolyl, hydroxyl, alkoxy or alkylthio each having up to 6 carbon atoms, carboxyl, alkoxycarbonyl having up to 6 carbon atoms, sulpho, alkylsulphonyl having up to 6 carbon atoms, phenylsulphonyl, tolylsulphonyl or by a group of the formula

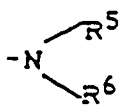

,

wherein

$R^5$ and $R^6$ are identical or different and denote hydrogen, alkyl having up to 8 carbon atoms, aryl having 6 to 12 carbon atoms, aralkyl having 7 to 14 carbon atoms, acyl having up to 7 carbon atoms,

alkylsulphonyl having up to 6 carbon atoms, phenylsulphonyl or tolylsulphonyl,

or wherein

$R^5$ and $R^6$ together with the nitrogen atom form a ring from the group comprising pyrrolidino, piperidino, piperazino, morpholino and thiomorpholino, it being possible for this ring to be substituted by alkyl having up to 4 carbon atoms or by phenyl,

$R^2$ represents straight-chain, branched or cyclic alkyl having up to 8 carbon atoms,

and

$R^3$ represents straight-chain or branched all having up to 4 carbon atoms,

and wherein the carbon atom 3 of the dihydropyrazine ring has the S-configuration and the carbon atom 6 has the R-configuration, the carbon atom 1' in the side chain has the S-configuration, if $R^1$ has a higher priority than the group $CH_2COOR^2$, or has the R-configuration, if $R^1$ has a lower priority than the group $CH_2COOR^2$, characterized in that compounds of the formula (III)

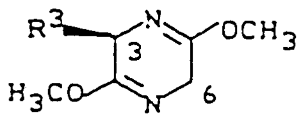

(III),

in which
$R^3$ has the meaning given above and the carbon atom of the dihydropyrazine ring has the R-configuration,
are first reacted with a strongly basic metal-organic compound to give the derivatives of (III) which are monosubstituted in the 6-position by the metal of the metal-organic compounds,
these metal derivatives are then reacted in inert solvents with a cis-substituted acrylic ester of the general formula (IV)

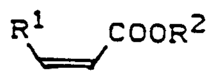

(IV),

in which
$R^1$ and $R^2$ have the meaning given above, and subsequently neutralized with an acid.

## Revendications

## Revendications pour les Etats contractants suivants : AT, BE, CH, DE, FR, GB, IT, LI, NL, SE

1. Enantiomères purs de γ-butyrolactames 4,5-disubstitués, de formule générale (I)

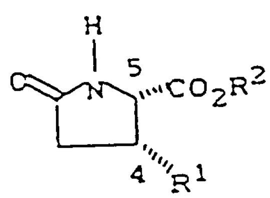

(I),

dans laquelle

$R^1$ représente un groupe aryle ayant 6 à 14 atomes de carbone, qui peut être substitué jusqu'à 5 fois identiques ou différentes par un radical alkyle, un radical alkoxy ou un radical alkylthio ayant chacun jusqu'à 8 atomes de carbone, par un radical aryle, un radical aryloxy, un radical arylthio ayant chacun 6 à 12 atomes de carbone, par un radical aralkyle ayant 7 à 14 atomes de carbone, par un halogène, un radical trifluorométhyle, trifluorométhoxy, difluorométhoxy, trifluorométhylthio, nitro, cyano, carboxy, alkoxycarbonyle ayant jusqu'à 8 atomes de carbone, sulfo, phénylsulfonyle, tolylsulfonyle, alkylsulfonyle ayant jusqu'à 8 atomes de carbone, hydroxy, ou par un groupe de formule

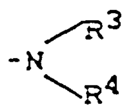 ,

dans laquelle

$R^3$ et $R^4$ sont identiques ou différents et représentent de l'hydrogène, un groupe alkyle ayant jusqu'à 8 atomes de carbone, un groupe aryle ayant 6 à 12 atomes de carbone, un groupe aralkyle ayant 7 à 14 atomes de carbone, un groupe acyle ayant jusqu'à 7 atomes de carbone, un groupe alkylsulfonyle ayant jusqu'à 6 atomes de carbone, un groupe phénylsulfonyle ou un groupe tolylsulfonyle,

ou bien

un noyau hétérocyclique de la série furyle, thiényle, pyridyle, pyrimidyle, pyrazinyle, pyridazinyle, quinolyle ou isoquinolyle, ces noyaux pouvant être substitués jusqu'à 3 fois identiques ou différentes par un radical alkyle, un radical alkoxy, un radical alkylthio ayant chacun jusqu'à 6 atomes de carbone, un halogène, un radical phényle, nitro, cyano ou par un groupe de formule

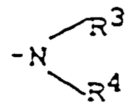 ,

dans laquelle

$R^3$ et $R^4$ ont la définition indiquée ci-dessus,

ou bien

un groupe alkyle ou un groupe alcényle linéaire, ramifié ou cyclique ayant jusqu'à 10 atomes de carbone, qui peut être substitué par un halogène, un radical aryle ayant 6 à 14 atomes de carbone, un radical furyle, thiényle, pyridyle, pyrimidyle, pyrazinyle, pyridazinyle, quinolyle, isoquinolyle, hydroxy, un radical alkoxy, alkylthio ayant chacun jusqu'à 6 atomes de carbone, carboxy, alkoxycarbonyle ayant jusqu'à 6 atomes de carbone, sulfo, alkylsulfonyle ayant jusqu'à 6 atomes de carbone, phénylsulfonyle, tolylsulfonyle, ou bien
par un groupe de formule

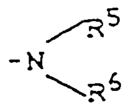 ,

dans laquelle

$R^5$ et $R^6$ sont identiques ou différents et représentent de l'hydrogène, un radical alkyle ayant jusqu'à 8 atomes de carbone, aryle ayant 6 à 12 atomes de carbone, aralkyle ayant 7 à 14 atomes de carbone, acyle ayant jusqu'à 7 atomes de carbone, alkylsulfonyle ayant jusqu'à 6 atomes de carbone, phénylsulfonyle ou tolylsulfonyle,

ou dans laquelle

$R^5$ et $R^6$ forment, conjointement avec l'atome d'azote, un noyau de la série pyrrolidino, pipéridino, pipérazino, morpholino ou thiomorpholino et ce noyau peut être subsstitué par un radical alkyle ayant jusqu'à 4 atomes de carbone ou par un radical phényle

$R^2$ est un groupe alkyle linéaire, ramifié ou cyclique ayant jusqu'à 8 atomes de carbone,

dans lesquels l'atome de carbone du noyau en position 5 a la configuration S et dans lesquels le substituant $R^1$ en position 4 par rapport au substituant $COOR^2$ en position 5 a la configuration cis.

**2.** Composés de formule générale (I) suivant la revendication 1, dans lesquels

$R^1$ représente un groupe phényle ou naphtyle, qui peut être substitué jusqu'à 3 fois identiques ou différentes par un radical alkyle, un radical alkoxy ayant chacun jusqu'à 6 atomes de carbone, un radical méthylthio, phényle, phénoxy, benzyle, fluoro, chloro, bromo, iodo, trifluorométhyle, trifluorométhoxy, difluorométhoxy, trifluorométhylthio, nitro, cyano, alkoxycarbonyle ayant jusqu'à 6 atomes de carbone, phénylsulfonyle, tolylsulfonyle, alkylsulfonyle ayant jusqu'à 6 atomes de carbone, hydroxy, ou bien
par un groupe de formule

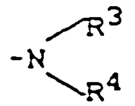 ,

dans laquelle

$R^3$ et $R^4$ sont identiques ou différents et représentent de l'hydrogène, un radical alkyle ayant jusqu'à 6 atomes de carbone, phényle, benzyle, acétyle, éthylcarbonyle, benzoyle, alkylsulfonyle ayant jusqu'à 4 atomes de carbone, tolylsulfonyle ou phénylsulfonyle,

ou bien
un noyau hétérocyclique de la série furyle, thiényle, pyridyle, pyrimidyle, quinolyle ou isoquinolyle, ces noyaux pouvant être substitués par un radical alkyle ou alkoxy ayant jusqu'à 4 atomes de carbone, par du fluor, du chlore, du brome, un radical nitro, cyano ou
par un groupe de formule

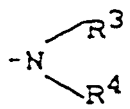 ,

dans laquelle

$R^3$ et $R^4$ ont la définition indiquée ci-dessus,

ou bien
un groupe alkyle linéaire, ramifié ou cyclique ayant jusqu'à 8 atomes de carbone, qui peut être substitué par du fluor, du chlore, du brome, un radical phényle, furyle, thiényle, pyridyle, pyrimidyle, quinolyle, isoquinolyle, hydroxy, un radical alkoxy ou alkylthio ayant jusqu'à 4 atomes de carbone, alkoxycarbonyle ayant jusuq'à 4 atomes de carbone, alkylsulfonyle ayant jusqu'à 4 atomes de carbone, phénylsulfonyle, tolylsulfonyle ou par un groupe de formule

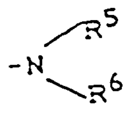 ,

dans laquelle

$R^5$ et $R^6$ sont identiques ou différents et représentent de l'hydrogène, un radical alkyle ayant jusqu'à 6 atomes de carbone, phényle, benzyle, acétyle, éthylcarbonyle, benzoyle, alkylsulfonyle ayant jusqu'à 4 atomes de carbone, tolylsulfonyle ou phénylsulfonyle,

ou dans laquelle

$R^5$ et $R^6$ forment, conjointement avec l'atome d'azote, un noyau de la série pyrrolidino, pipéridino, N-méthyl- ou N-phénylpipérazino ou morpholino

et

$R^2$ est un groupe alkyle linéaire ou ramifié ayant jusqu'à 6 atomes de carbone,

et dans lesquels l'atome de carbone du noyau en position 5 a la configuration S et dans lesquels le substituant $R^1$ en position 4 par rapport au substituant $COOR^2$ en position 5 a la configuration cis.

**3.** Composés de formule générale (I) suivant la revendication 1, dans laquelle

$R^1$ représente un groupe phényle qui peut être substitué par un radical alkyle ou un radical alkoxy ayant jusqu'à 4 atomes de carbone, par du fluor, du chlore, du brome, un radical trifluorométhyle, trifluorométhoxy, trifluorométhylthio, nitro, cyano, alkoxycarbonyle ayant jusqu'à 4 atomes de carbone, phénylsulfonyle, tolylsulfonyle, alkylsulfonyle ayant jusqu'à 4 atomes de carbone, hydroxy, ou par un groupe de formule

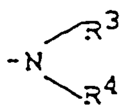 ,

dans laquelle

$R^3$ et $R^4$ sont identiques ou différents et représentent de l'hydrogène, un radical phényle, benzyle ou acétyle,

ou bien

- un groupe furyle, thiényle ou pyridyle, ou bien
- un groupe alkyle linéaire, ramifié ou cyclique ayant jusqu'à 6 atomes de carbone, qui peut être substitué par du fluor, du chlore, du brome, un radical phényle, thiényle, pyridyle, furyle ou alkoxy ayant jusqu'à 4 atomes de carbone,

et

$R^2$ est un groupe alkyle linéaire ou ramifié ayant jusqu'à 4 atomes de carbone,

dans lesquels l'atome de carbone du noyau en position 5 a la configuration S et dans lesquels le substituant $R^1$ en position 4 par rapport au substituant $COOR^2$ en position 5 a la configuration cis.

**4.** Procédé de production d'énantiomères purs de γ-butyrolactames 4,5-disubstitués de formule générale (I) dans laquelle

$R^1$ représente un groupe aryle ayant 6 à 12 atomes de carbone, qui peut être substitué jusqu'à 5 fois identiques ou différentes par un radical alkyle, un radical alkoxy ou un radical alkylthio ayant chacun jusqu'à 8 atomes de carbone, par un radical aryle, un radical aryloxy, un radical arylthio ayant chacun 6 à 12 atomes de carbone, par un radical aralkyle ayant 7 à 14 atomes de carbone, par un halogène, un radical trifluorométhyle, trifluorométhoxy, difluorométhoxy, trifluorométhylthio, nitro, cyano, carboxy, alkoxycarbonyle ayant jusqu'à 8 atomes de carbone, sulfo, phénylsulfonyle, tolylsulfonyle, alkylsulfonyle ayant jusqu'à 8 atomes de carbone, hydroxy, ou par un groupe de formule

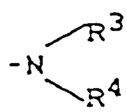 ,

dans laquelle

$R^3$ et $R^4$ sont identiques ou différents et représentent de l'hydrogène, un groupe alkyle ayant jusqu'à 8 atomes de carbone, un groupe aryle ayant 6 à 12 atomes de carbone, un groupe aralkyle ayant 7 à 14 atomes de carbone, un groupe acyle ayant 2 à 7 atomes de carbone, un groupe alkylsulfonyle ayant jusqu'à 6 atomes de carbone, un groupe phénylsulfonyle ou un groupe tolylsulfonyle,

ou bien

un noyau hétérocyclique de la série furyle, thiényle, pyridyle, pyrimidyle, pyrazinyle, pyridazinyle, quinolyle ou isoquinolyle, ces noyaux pouvant être substitués jusqu'à 3 fois identiques ou différentes par un radical alkyle, un radical alkoxy, un radical alkylthio ayant chacun jusqu'à 6 atomes de carbone, un halogène, un radical phényle, nitro, cyano ou par un groupe de formule

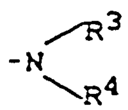 ,

dans laquelle

$R^3$ et $R^4$ ont la définition indiquée ci-dessus,

ou bien

un groupe alkyle ou un groupe alcényle linéaire, ramifié ou cyclique ayant jusqu'à 10 atomes de carbone, qui peut être substitué par un halogène, un radical aryle ayant 6 à 12 atomes de carbone, un radical furyle, thiényle, pyridyle, pyrimidyle, pyrazinyle, pyridazinyle, quinolyle, isoquinolyle, hydroxy, un radical alkoxy, alkylthio ayant chacun jusqu'à 6 atomes de carbone, carboxy, alkoxycarbonyle ayant jusqu'à 6 atomes de carbone, sulfo, alkylsulfonyle ayant jusqu'à 6 atomes de carbone, phénylsulfonyle, tolylsulfonyle, ou bien
par un groupe de formule

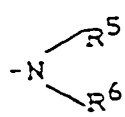 ,

dans laquelle

$R^5$ et $R^6$ sont identiques ou différents et représentent de l'hydrogène, un radical alkyle ayant jusqu'à 8 atomes de carbone, aryle ayant 6 à 12 atomes de carbone, aralkyle ayant 7 à 14 atomes de carbone, acyle ayant jusqu'à 7 atomes de carbone, alkylsulfonyle ayant jusqu'à 6 atomes de carbone, phénylsulfonyle ou tolylsulfonyle,

ou dans laquelle

$R^5$ et $R^6$ forment, conjointement avec l'atome d'azote, un noyau de la série pyrrolidino, pipéridino, pipérazino, morpholino ou thiomorpholino et ce noyau peut être substitué par un radical alkyle ayant jusqu'à 4 atomes de carbone ou par un radical phényle

et

$R^2$ est un groupe alkyle linéaire, ramifié ou cyclique ayant jusqu'à 8 atomes de carbone,

dans lesquels l'atome de carbone du noyau en position 5 a la configuration S et dans lesquels le substituant $R^1$ en position 4 par rapport au substituant $COOR^2$ en position 5 a la configuration cis, caractérisé en ce qu'on hydrolyse tout d'abord dans des solvants inertes, avec des acides, des dihydropyrazines de formule générale (II)

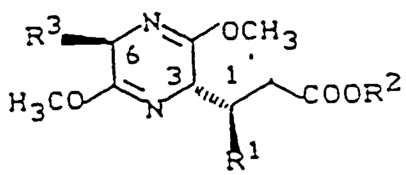

(II)

dans laquelle

$R^1$ et $R^2$ ont la définition indiquée ci-dessus,

et

$R^3$ est un groupe alkyle linéaire ou ramifié ayant jusqu'à 4 atomes de carbone,

et dans lequel l'atome de carbone en position 6 du noyau de dihydropyrazine dans le composé (II) a la configuration R, l'atome de carbone en position 3 du noyau de pyrazine dans le composé (II) a la configuration S et l'atome de carbone en position 1' a la configuration S, lorsque $R^1$ a une priorité plus haute que le groupe $CH_2COOR^2$, ou la configuration R lorsque $R^1$ a une priorité plus basse que le groupe $CH_2COOR^2$,

puis, à partir des sels acides d'amino-acides formés, on prépare avec des bases, dans des solvants inertes, les amino-acides libres

et on cyclise ensuite ces derniers.

**5.** Dihydropyrazines de formule générale (II)

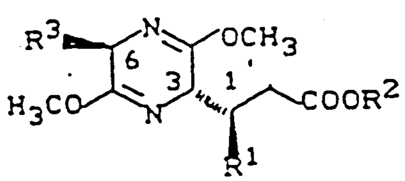

(II)

dans laquelle

$R^1$ est un groupe aryle ayant 6 à 14 atomes de carbone, qui peut être substitué jusqu'à 5 fois identiques ou différentes par un groupe alkyle, un groupe alkoxy, un groupe alkylthio ayant chacun jusqu'à 8 atomes de carbone, par un groupe aryle, un groupe aryloxy, un groupe arylthio ayant chacun 6 à 12 atomes de carbone, par un groupe aralkyle ayant 7 à 14 atomes de carbone, par un halogène, un groupe trifluorométhyle, trifluorométhoxy, difluorométhoxy, trifluorométhylthio, nitro, cyano, carboxy, alkoxycarbonyle ayant jusqu'à 8 atomes de carbone, sulfo, phénylsulfonyle, tolylsulfonyle, alkylsulfonyle ayant jusqu'à 8 atomes de carbone,

hydroxy, ou par un groupe de formule

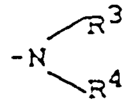

dans laquelle

R$^3$ et R$^4$ sont identiques ou différents et représentent de l'hydrogène, un groupe alkyle ayant jusqu'à 8 atomes de carbone, un groupe aryle ayant 6 à 12 atomes de carbone, un groupe aralkyle ayant 7 à 14 atomes de carbone, un groupe acyle ayant jusqu'à 7 atomes de carbone, un groupe alkylsulfonyle ayant jusqu'à 6 atomes de carbone, un groupe phénylsulfonyle ou un groupe tolylsulfonyle,

ou bien

un noyau hétérocyclique de la série furyle, thiényle, pyridyle, pyrimidyle, pirazinyle, pyridazinyle, quinolyle ou isoquinolyle, ces noyaux pouvant être substitués jusqu'à 3 fois identiques ou différentes par un groupe alkyle, un groupe alkoxy, un groupe alkylthio ayant chacun jusqu'à 6 atomes de carbone, un halogène, un groupe phényle, nitro, cyano ou par un groupe de formule

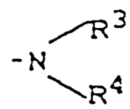 ,

dans laquelle

R$^3$ et R$^4$ ont la définition indiquée ci-dessus,

ou bien

un groupe alkyle ou un groupe alcényle linéaire, ramifié ou cyclique ayant jusqu'à 10 atomes de carbone, qui peut être substitué par un halogène, un radical aryle ayant 6 à 12 atomes de carbone, furyle, thiényle, pyridyle, pyrimidyle, pyrazinyle, pyridazinyle, quinolyle, isoquinolyle, hydroxy, un radical alkoxy, un radical alkylthio ayant chacun jusqu'à 6 atomes de carbone, carboxy, un radical alkoxycarbonyle ayant jusqu'à 6 atomes de carbone, sulfo, alkylsulfonyle ayant jusqu'à 6 atomes de carbone, phénylsulfonyle, tolylsulfonyle, ou bien par un groupe de formule

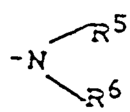 ,

dans laquelle

R$^5$ et R$^6$ sont identiques ou différents et représentent de l'hydrogène, un radical alkyle ayant jusqu'à 8 atomes de carbone, aryle ayant 6 à 12 atomes de carbone, aralkyle ayant 7 à 14 atomes de carbone, acyle ayant jusqu'à 7 atomes de carbone, alkylsulfonyle ayant jusqu'à 6 atomes de carbone, phénylsulfonyle ou tolylsulfonyle,

ou dans laquelle

R$^5$ et R$^6$ forment, conjointement avec l'atome d'azote, un noyau de la série pyrrolidino, pipéridino, pipérazino, morpholino ou thiomorpholino et ce noyau peut être substitué par un radical alkyle ayant jusqu'à 4 atomes de carbone ou par un radical phényle,

$R^2$ est un groupe alkyle linéaire, ramifié ou cyclique ayant jusqu'à 8 atomes de carbone,

et

$R^3$ est un radical alkyle linéaire ou ramifié ayant jusqu'à 4 atomes de carbone,

et dans laquelle l'atome de carbone en position 3 du noyau de dihydropyrazine a la configuration S et l'atome de carbone en position 6 a la configuration R, l'atome de carbone en position 1' dans la chaîne latérale a la configuration S lorsque $R^1$ a une priorité plus haute que le groupe $CH_2COOR^2$, ou la configuration R lorsque $R^1$ a une priorité plus basse que le groupe $CH_2COOR^2$.

6. Procédé de production de dihydropyrazines de formule générale II suivant la revendication 5, caractérisé en ce qu'on fait réagir tout d'abord avec un composé organométallique fortement basique, des composés de formule (III)

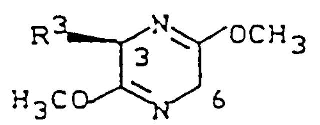

(III),

dans laquelle
$R^3$ a la définition indiquée ci-dessus et l'atome de carbone du noyau de dihydropyrazine a la configuration R, pour former les dérivés de (III) monosubstitués en position 6 par le métal des composés organométalliques, puis on fait réagir ces dérivés métalliques dans des solvants inertes avec un ester acrylique à substitution cis, de formule générale (IV)

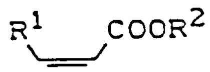

(IV),

dans laquelle
$R^1$ et $R^2$ ont la définition indiquée ci-dessus, et on neutralise ensuite avec un acide.

**Revendications pour l'Etat contractant suivant : ES**

1. Procédé de production d'énantiomères purs de $\gamma$-butyrolactames 4,5-disubstitués de formule générale (I)

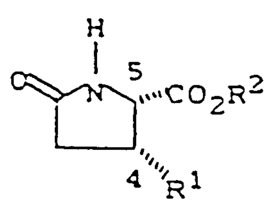

(I),

dans laquelle

$R^1$ représente un groupe aryle ayant 6 à 14 atomes de carbone, qui peut être substitué jusqu'à 5 fois identiques ou différentes par un radical alkyle, un radical alkoxy ou un radical alkylthio ayant chacun jusqu'à 8 atomes de carbone, par un radical aryle, un radical aryloxy, un radical arylthio ayant chacun 6 à 12 atomes de carbone, par un radical alkyle ayant 7 à 14 atomes de carbone, par un halogène, un radical trifluorométhyle, trifluorométhoxy, difluorométhoxy, trifluorométhylthio, nitro, cyano, carboxy, alkoxycarbonyle ayant jusqu'à 8 atomes de carbone, sulfo, phénylsulfonyle, tolylsulfonyle, alkylsulfonyle ayant jusqu'à 8 atomes de carbone, hydroxy, ou par un groupe de formule

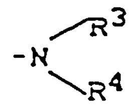 ,

dans laquelle

$R^3$ et $R^4$ sont identiques ou différents et représentent de l'hydrogène, un groupe alkyle ayant jusqu'à 8 atomes de carbone, un groupe aryle ayant 6 à 12 atomes de carbone, un groupe aralkyle ayant 7 à 14 atomes de carbone, un groupe acyle ayant jusqu'à 7 atomes de carbone, un groupe alkylsulfonyle ayant jusqu'à 6 atomes de carbone, un groupe phénylsulfonyle ou un groupe tolylsulfonyle,

ou bien

un noyau hétérocyclique de la série furyle, thiényle, pyridyle, pyrimidyle, pyrazinyle, pyridazinyle, quinolyle ou isoquinolyle, ces noyaux pouvant être substitués jusqu'à 3 fois identiques ou différentes par un radical alkyle, un radical alkoxy, un radical alkylthio ayant chacun jusqu'à 6 atomes de carbone, un halogène, un radical phényle, nitro, cyano ou par un groupe de formule

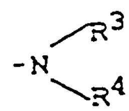 ,

dans laquelle

$R^3$ et $R^4$ ont la définition indiquée ci-dessus,

ou bien

un groupe alkyle ou un groupe alcényle linéaire, ramifié ou cyclique ayant jusqu'à 10 atomes de carbone, qui peut être substitué par un halogène, un radical aryle ayant 6 à 14 atomes de carbone, un radical furyle, thiényle, pyridyle, pyrimidyle, pyrazinyle, pyridazinyle, quinolyle, isoquinolyle, hydroxy, un radical alkoxy, alkylthio ayant chacun jusqu'à 6 atomes de carbone, carboxy, alkoxycarbonyle ayant jusqu'à 6 atomes de carbone, sulfo, alkylsulfonyle ayant jusqu'à 6 atomes de carbone, phénylsulfonyle, tolylsulfonyle, ou bien
par un groupe de formule

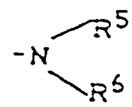 ,

dans laquelle

$R^5$ et $R^6$ sont identiques ou différents et représentent de l'hydrogène, un radical alkyle ayant jusqu'à 8 atomes de carbone, aryle ayant 6 à 12 atomes de carbone, aralkyle ayant 7 à 14 atomes de carbone, acyle ayant jusqu'à 7 atomes de carbone, alkylsulfonyle ayant jusqu'à 6 atomes de carbone, phénylsulfonyle ou tolylsulfonyle,

ou dans laquelle

$R^5$ et $R^6$ forment, conjointement avec l'atome d'azote, un noyau de la série pyrrolidino, pipéridino, pipérazino, morpholino ou thiomorpholino et ce noyau peut être substitué par un radical alkyle ayant jusqu'à 4 atomes de carbone ou par un radical phényle

$R^2$ est un groupe alkyle linéaire, ramifié ou cyclique ayant jusqu'à 8 atomes de carbone,

dans lesquels l'atome de carbone du noyau en position 5 a la configuration S et dans lesquels le substituant $R^1$ en position 4 par rapport au substituant $COOR^2$ en position 5 a la configuration cis, caractérisé en ce qu'on hydrolyse

tout d'abord dans des solvants inertes, avec des acides, des dihydropyrazines de formule générale (II)

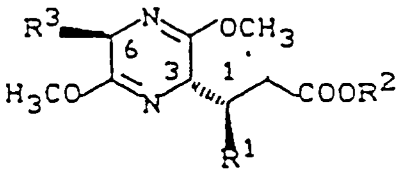

(II)

dans laquelle

$R^1$ et $R^2$ ont la définition indiquée ci-dessus,

et

$R^3$ est un groupe alkyle linéaire ou ramifié ayant jusqu'à 4 atomes de carbone,

et dans lequel l'atome de carbone en position 6 du noyau de dihydropyrazine dans le composé (II) a la configuration R, l'atome de carbone en position 3 du noyau de pyrazine dans le composé (II) a la configuration S et l'atome de carbone en position 1' a la configuration S, lorsque $R^1$ a une priorité plus haute que le groupe $CH_2COOR^2$, ou la configuration R lorsque $R^1$ a une priorité plus basse que le groupe $CH_2COOR^2$,

puis, à partir des sels acides d'amino-acides formés, on prépare avec des bases, dans des solvants inertes, les amino-acides libres

et on cyclise ensuite ces derniers.

**2.** Procédé de production de dihydropyrazines de formule générale (II)

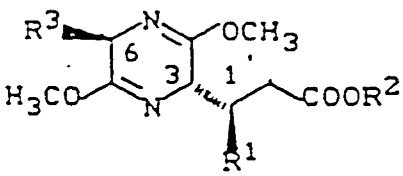

(II)

dans laquelle

$R^1$ est un groupe aryle ayant 6 à 14 atomes de carbone, qui peut être substitué jusqu'à 5 fois identiques ou différentes par un groupe alkyle, un groupe alkoxy, un groupe alkylthio ayant chacun jusqu'à 8 atomes de carbone, par un groupe aryle, un groupe aryloxy, un groupe arylthio ayant chacun 6 à 12 atomes de carbone, par un groupe aralkyle ayant 7 à 14 atomes de carbone, par un halogène, un groupe trifluorométhyle, trifluorométhoxy, difluorométhoxy, trifluorométhylthio, nitro, cyano, carboxy, alkoxycarbonyle ayant jusqu'à 8 atomes de carbone, sulfo, phénylsulfonyle, tolylsulfonyle, alkylsulfonyle ayant jusqu'à 8 atomes de carbone, hydroxy, ou par un groupe de formule

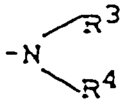

dans laquelle

$R^3$ et $R^4$ sont identiques ou différents et représentent de l'hydrogène, un groupe alkyle ayant jusqu'à 8 atomes de carbone, un groupe aryle ayant 6 à 12 atomes de carbone, un groupe aralkyle ayant 7 à

14 atomes de carbone, un groupe acyle ayant jusqu'à 7 atomes de carbone, un groupe alkylsulfonyle ayant jusqu'à 6 atomes de carbone, un groupe phénylsulfonyle ou un groupe tolylsulfonyle,

ou bien

un noyau hétérocyclique de la série furyle, thiényle, pyridyle, pyrimidyle, pirazinyle, pyridazinyle, quinolyle ou isoquinolyle, ces noyaux pouvant être substitués jusqu'à 3 fois identiques ou différentes par un groupe alkyle, un groupe alkoxy, un groupe alkylthio ayant chacun jusqu'à 6 atomes de carbone, un halogène, un groupe phényle, nitro, cyano ou par un groupe de formule

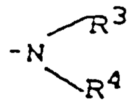

dans laquelle

$R^3$ et $R^4$ ont la définition indiquée ci-dessus,

ou bien

un groupe alkyle ou un groupe alcényle linéaire, ramifié ou cyclique ayant jusqu'à 10 atomes de carbone, qui peut être substitué par un halogène, un radical aryle ayant 6 à 12 atomes de carbone, furyle, thiényle, pyridyle, pyrimidyle, pyrazinyle, pyridazinyle, quinolyle, isoquinolyle, hydroxy, un radical alkoxy, un radical alkylthio ayant chacun jusqu'à 6 atomes de carbone, carboxy, un radical alkoxycarbonyle ayant jusqu'à 6 atomes de carbone, sulfo, alkylsulfonyle ayant jusqu'à 6 atomes de carbone, phénylsulfonyle, tolylsulfonyle, ou bien par un groupe de formule

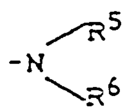 ,

dans laquelle

$R^5$ et $R^6$ sont identiques ou différents et représentent de l'hydrogène, un radical alkyle ayant jusqu'à 8 atomes de carbone, aryle ayant 6 à 12 atomes de carbone, aralkyle ayant 7 à 14 atomes de carbone, acyle ayant jusqu'à 7 atomes de carbone, alkylsulfonyle ayant jusqu'à 6 atomes de carbone, phénylsulfonyle ou tolylsulfonyle,

ou dans laquelle

$R^5$ et $R^6$ forment, conjointement avec l'atome d'azote, un noyau de la série pyrrolidino, pipéridino, pipérazino, morpholino ou thiomorpholino et ce noyau peut être subtitué par un radical alkyle ayant jusqu'à 4 atomes de carbone ou par un radical phényle,

$R^2$ est un groupe alkyle linéaire, ramifié ou cyclique ayant jusqu'à 8 atomes de carbone,

et

$R^3$ est un radical alkyle linéaire ou ramifié ayant jusqu'à 4 atomes de carbone,

et dans laquelle l'atome de carbone en position 3 du noyau de dihydropyrazine a la configuration S et l'atome de carbone en position 6 a la configuration R, l'atome de carbone en position 1' dans la chaîne latérale a la configuration S lorsque $R^1$ a une priorité plus haute que le groupe $CH_2COOR^2$, ou la configuration R lorsque $R^1$ a une priorité plus basse que le groupe $CH_2COOR^2$,

caractérisé en ce qu'on fait réagir tout d'abord avec un composé organométallique fortement basique, des composés de formule (III)

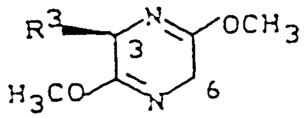

(III),

dans laquelle

$R^3$ a la définition indiquée ci-dessus et l'atome de carbone du noyau de dihydropyrazine a la configuration R,

pour former les dérivés de (III) monosubstitués en position 6 par le métal des composés organométalliques,

puis on fait réagir ces dérivés métalliques dans des solvants inertes avec un ester acrylique à substitution cis, de formule générale (IV)

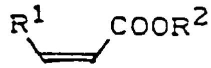

(IV),

dans laquelle

$R^1$ et $R^2$ ont la définition indiquée ci-dessus, et on neutralise ensuite avec un acide.